# EUROPÄISCHE PATENTSCHRIFT

(11) **EP 1 131 393 B1**
(45) Veröffentlichungstag und Bekanntmachung des Hinweises auf die Patenterteilung: **11.08.2004**
(21) Anmeldenummer: 00965842.8
(22) Anmeldetag: 13.09.2000
(51) Int. Cl.: A61K 7/50, C11D 1/32, C11D 1/28, C11D 1/10, C11D 1/34, C11D 1/04, C11D 1/90, C11D 1/52, C11D 1/94, C11D 1/83

(54) **TENSIDZUSAMMENSETZUNG ENTHALTEND GEMINITENSIDE UND DEREN VERWENDUNG ZUR HAUT- UND HAARREINIGUNG**
TENSIDE COMPOSITION CONTAINING GEMINI TENSIDES AND USE THEREOF FOR CLEANING SKIN AND HAIR
COMPOSITION TENSIOACTIVE CONTENANT DES TENSIOACTIFS GEMINI ET SON UTILISATION POUR LE NETTOYAGE DE LA PEAU ET DES CHEVEUX

(30) Priorität: 13.09.1999 DE 19943681
(43) Veröffentlichungstag der Anmeldung: 12.09.2001
(73) Patentinhaber: SASOL Germany GmbH, 22297 Hamburg (DE)
(72) Erfinder: DAHMS, Gerd, H., 47138 Duisburg (DE); KWETKAT, Klaus, 59192 Bergkamen (DE)
(74) Vertreter: Schupfner, Georg U.
(86) Internationale Anmeldenummer: PCT/DE2000/003163
(87) Internationale Veröffentlichungsnummer: WO 2001/019943

(56) Entgegenhaltungen:
- EP-A- 0 697 244
- EP-A- 0 697 245
- EP-A- 0 884 298
- WO-A-97/40124
- WO-A-98/20853
- DE-A- 19 750 245
- DE-A- 19 750 246
- DE-A- 19 855 080
- US-A- 5 863 886
- ROBERT R. LINTON, R.I.T.A. CORP. CRYSTAL LAKE, IL : "Acyl lactylates in cosmetics" DRUG COSMET.IND., Bd. 134, Nr. 5, - Mai 1984 (1984-05) Seiten 52-57, XP000926157

## Beschreibung

Die vorliegende Erfindung betrifft eine Tensidzusammensetzung, die ein oder mehrere Geminitenside und zumindest eine andersartige Detergens-Komponente mit milder, schlecht-anschäumender Charakteristik enthält, und deren Einsatz als multifunktionelle kosmetische Zubereitung zur Haut- und Haarreinigung bzw. -pflege.

Um Hautschädigungen durch tägliches Reinigen von Haut und Haar weitgehendst auszuschließen, ist es unerläßlich, Detergentien zum Einsatz zu bringen, die als mild und nicht hautirritierend eingestuft werden. Es ist allerdings bekannt, daß mit zunehmender Hautverträglichkeit das Schäumvermögen von Tensiden - als einer der Basiskomponenten eines Detergens - derart stark nachläßt, daß ein ausreichendes Anschäumen derartiger Tenside auf der Haut oder dem Haar und somit dessen weitflächige Verteilung und die an sich beabsichtigte Schmutzaufnahme nicht gewährleistet ist. Werden solche Tenside dennoch in Detergentien zum Einsatz gebracht, müssen sie oftmals mit weitaus weniger milden Tensiden kombiniert werden.

Geminitenside werden seit einiger Zeit untersucht und weisen gegenüber der Haut eine außerordentliche Mildheit auf. Aufgrund ihrer geringen Schäumbarkeit konnten sie jedoch bislang nicht in gewünschtem Ausmaß erfolgreich in Reinigungsprodukten eingesetzt werden. Einen guten Überblick über den Stand der Technik auf dem Gebiet der Geminitenside gibt R. Zana "Bolaform and dimeric (gemini) surfactants" in Novel Surfactants: Preparation, Application and Biogradability, C. Holmberg (ed.), Marcel Dekker, (1998), 81-103.

In der EP-A-0 697 244 werden amphotere Geminitenside beschrieben, die auch als Mischung mit anderen anionischen, nichtionischen, kationischen oder amphoteren Tensiden eingesetzt werden können. Als Verwendung ist der Einsatz in Reinigungsmitteln offenbart.

Aus der WO 95/19953 sind Geminitenside (Geminiamide) bekannt, die als Komponente u.a. in üblichen Reinigungsmittel-Zusammensetzungen verwendet werden können. Die WO 95/19955 nennt Geminipolyether als eine weitere Klasse von Geminitensiden, die ebenfalls auf dem vorstehenden Anwendungsgebiet eingesetzt werden können. Gemische von Geminitensiden des alkoxylierten Bisalkylphenol-Typs mit anderen Tensiden sind aus der WO 97/23449 bekannt.

Gemische von Geminitensiden des alkoxylierten Bisalkylphenol-Typs mit anderen Tensiden sind aus der WO 97/23449 bekannt. In der JP-A 11060430 und JP-A 11060437 wird der Einsatz von anionischen Geminitensiden in Kosmetika beschrieben, wobei diese auch gemeinsam mit anderen Tensiden vorliegen können.

Aufgabe der vorliegenden Erfindung ist es, Tensidmischungen bereitzustellen, die es ermöglichen, die dermatologischen Vorteile der Geminitenside zu erhalten und gleichzeitig die Schäumbarkeit zu verbessern und damit die Reinigungskraft der Tensidzusammensetzungen so zu erhöhen, daß diese herkömmlichen Reinigungsmitteln für die Haar- und Hautreinigung überlegen sind.

Diese Aufgabe wird erfindungsgemäß gelöst durch eine Tensidzusammensetzung **gemäß Anspruch 1**.

Vorteilhafte Ausführungsformen der Erfindung sind Gegenstand der Unteransprüche.

Überraschend hat sich herausgestellt, daß die erfindungsgemäße Tensidzusammensetzung zu einem außerordentlich cremigen und feinperligen Naßschaum mit sehr guter Anschäumbarkeit führt, wie dies für die Haar- und Hautreinigung erforderlich ist. Neben dem guten Schäumvermögen zeichnen sich derartige Tensidmischungen bei der Anwendung auf dem Haar aufgrund der hohen Affinität der Geminitenside durch eine erhebliche Verbesserung der Naß- und Trockenkämmbarkeit bedingt durch eine gute antistatische Wirkung aus. Bei der Hautreinigung werden ein sehr angenehmes seidiges Hautgefühl und gute rückfettende Eigenschaften beobachtet. Die erfindungsgemäßen Mischungen zeichnen sich dadurch aus, daß sie das Irritationspotential gängiger anionischer Tenside deutlich herabsetzen. So läßt sich durch Zusatz der erfindungsgemäßen Tensidzusammensetzungen zu Alkylethersulfaten, Alkylbenzolsulfonaten und anderen anionischen Tensiden deren Irritationspotential signifikant herabsetzen.

Unter Geminitensiden versteht man - im Rahmen der vorliegenden Erfindung - grenzflächenaktive Verbindungen, die aus mindestens zwei, vorzugsweise aus zwei, Tensideinheiten, d.h. aus hydrophiler Kopfgruppe und hydrophober Gruppe, bestehen, die durch mindestens einen, vorzugsweise durch einen, Abstandhalter, Spacer genannt, in der Nähe der Kopfgruppe miteinander verknüpft sind. Die Gemini- oder Zwillingstenside werden auch Dimertenside genannt und tragen ihren Namen aufgrund ihrer besonderen Bauart. Abhängig von der Natur der Kopfgruppe gibt es anionische, nichtionische, kationische und amphotere Geminitenside. Gegestand der Erfindung sind anionische, kationische und neutrale Geminitenside. Anders als konventionelle Tenside, die man ebenfalls in diese Gruppen einteilt, können Geminitenside jedoch auch Kombinationen von Kopfgruppen unterschiedlichen Charakters tragen. Dabei handelt. es sich meist um Kombinationen aus nichtionischen und ionischen Gruppen.

Bei Kombinationen von ionischen mit nichtionischen Kopfgruppen überwiegt die Natur der ionischen Kopfgruppe im resultierenden Geminitensid, so daß Kombinationen aus nichtionischer und anionischer Kopfgruppe als anionisches Geminitensid klassifiziert werden. Analoges gilt für die Kombinationen nichtionischer mit kationischen oder amphoteren Kopfgruppen. Für die erfindungsgemäßen Tensidzusammensetzungen ist in erster Linie die Morphologie, also die relative Anordnung der verschiedenen Struktureinheiten (hydrophile Gruppen, Spacer, hydrophobe Ketten) zueinander, entscheidend und nicht die Art der Kopfgruppe. Die erfindungsgemäß eingesetzten Geminitenside weisen demnach folgende Struktur auf:

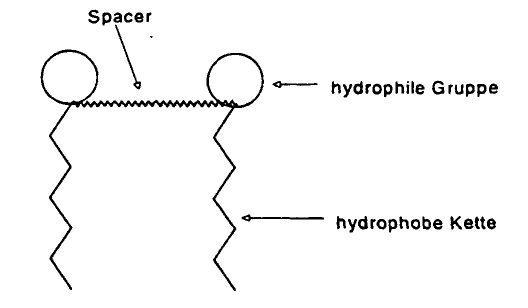

Zu den bevorzugt in den erfindungsgemäßen Tensidzusammensetzungen eingesetzten Geminitensiden zählen solche, die an der Verknüpfungsstelle zwischen Spacer, hydrophiler und hydrophober Gruppe Stickstoffatome enthalten. Dazu gehören bevorzugt Geminitenside mit Amin- oder Amidgruppen aufweisenden Spacern aber auch sich von Dicarbonsäuren ableitende Spacer, sich von Betainstrukturen ableitende, hydrophile Doppelkopfgruppen, die gegebenenfalls durch Alkoxylieren (insbesondere Ethoxylieren) hergestellte Seitengruppen aufweisen, die Sulfonsäure-, Phosphonsäure-, Carbonsäure oder Alkoholgruppen (einschließlich Polyalkoholen) tragen können, und hydrophobe Doppelketten mit 5 bis 25 C-Atomen, die verzweigt oder unverzweigt sein können und bis zu 2 nicht benachbarte Doppelbindungen tragen können.

Die folgenden Strukturvarianten der Geminitenside sind für die erfindungsgemäße Tensidzusammensetzungen besonders geeignet.

### Variante A: Auf amid- oder aminhaltigen Spacern beruhende Strukturen

### A.I Geminitenside der allgemeinen Formel (A.I) analog WO 96/14926

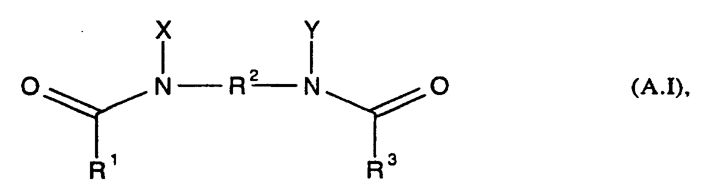

wobei die Substituenten folgende Bedeutung haben:
- R¹, R³: C₅- bis C₂₅-Alkyl, verzweigt oder unverzweigt, gesättigt, gegebenenfalls bis zu 2-fach nicht-benachbart ungesättigt;
- R²: C₁- bis C₁₂-Alkylen;
- X, Y: (C₂H₄O-)ₓ(C₃H₆O-)_{y}-FR; x+y ≥ 1, x: 0-15, y: 0-10 und
- FR: -SO₃M, -CH₂-CO₂M, -P(O)(OM)₂, H, -C₃H₆SO₃M; oder -CH₂(CHOH)₄CH₂OH, soweit x+y=0; wobei M = Alkali, (Alkyl)Ammonium, Alkanolammonium, H oder ½ Erdalkali ist.

### A.II Geminitenside mit Dicarbonsäure-stämmigen Spacern der allgemeinen Formel (A.II) analog WO 96/25388

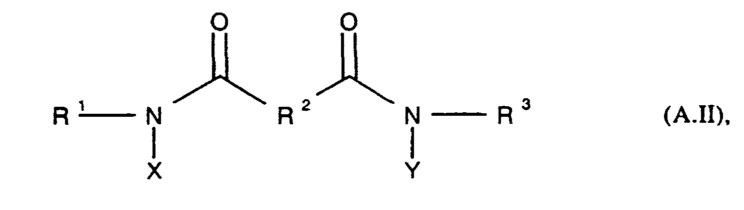

wobei die Substituenten die für die allgemeine Formel (A.I) angegebene Bedeutung haben.

### A.III Amphotere Geminitenside der allgemeinen Formel (A.III) analog WO 97/31890

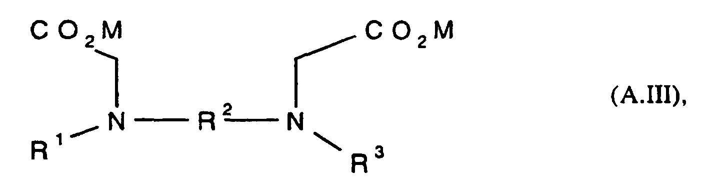

wobei die Substituenten die für die allgemeine Formel (A.I) angegebene Bedeutung haben. Geminitenside der allgemeinen Formel (A.III) sind amphotere Verbindungen, so daß sie bei entsprechend saurem Umgebungsmedium auch kationisch werden können.

### Variante B: Auf amid- oder aminhaltigen Spacern beruhende Strukturen

### B.I Geminitenside der allgemeinen Formel (B.I) analog DE 19622612 oder JP-A 10-175934

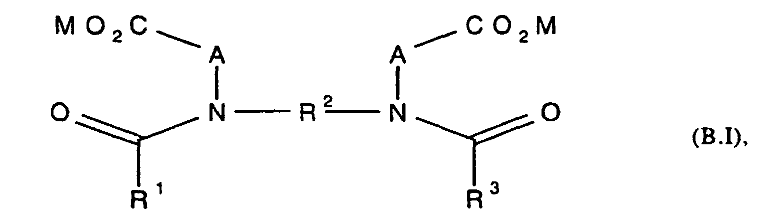

wobei die Substituenten folgende Bedeutung haben:
- R¹, R³: C₅- bis C₂₅- Alkyl, verzweigt oder unverzweigt, gesättigt, gegebenenfalls bis zu 2-fach nicht-benachbart ungesättigt;
- R²: C₁- bis C₁₂-Alkylen;
- A: CHR⁴, CH₂, C₂H₄, C₃H₆, C₄H₈;
- R⁴: Rest einer Aminocarbonsäure und
- M: Alkali, (Alkyl)Ammonium, Alkanolammonium, H oder ½ Erdalkali.

### B.II Geminitenside der allgemeinen Formel (B.II) analog EP 0 708 079

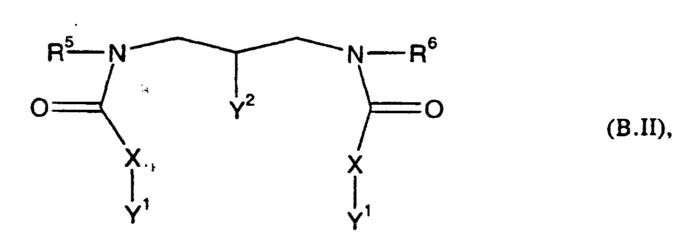

wobei die Substituenten die für die allgemeine Formel (B.I) angegebene Bedeutung haben und
- R⁵, R⁶: C₆- bis C₃₆-Alkyl, verzweigt oder unverzweigt, gesättigt, gegebenenfalls bis zu 2-fach nicht-benachbart ungesättigt;
- X: Alkylen- oder Alkenylengruppe mit 1 bis 6 Kohlenstoffatomen, die mit einer Hydroxylgruppe oder einer Sulfonsäuregruppe oder einer Carboxylgruppe substituiert sein kann;
- Y¹: eine Sulfonat- oder Sulfatgruppe oder eine Carboxylgrupppe und
- Y²: eine Hydroxylgruppe, ein Schwefelsäurerest oder -O-(CO)X-COOH bedeuten.

### B.III Geminitenside der allgemeinen Formel (B.III) analog JP-A-8-311003

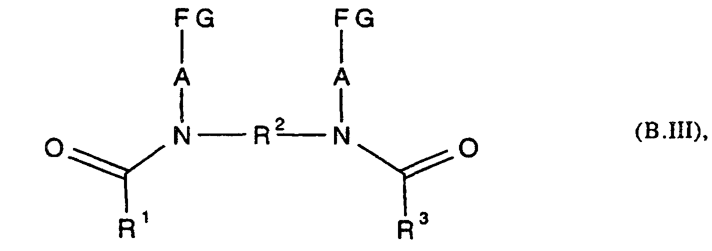

wobei die Substituenten die für die allgemeine Formel (B.I) angegebene Bedeutung haben und
- FG: -COOM oder -SO₃M bedeutet.

### B.IV Geminitenside der allgemeinen Formel (B.IV) analog JP-A 11-60437

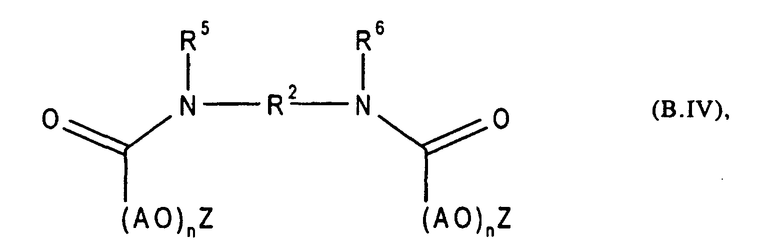

wobei die Substituenten, die bei den allgemeinen Formeln (B.I) und (B.II) angegebene Bedeutung haben und
- AO: Alkylenoxideinheiten, d.h. Ethylenglykol-, Propylenglykol und Butylenglykolethereinheiten, allein oder statistisch oder blockweise verteilt, mit n = 1 bis 20 und
- Z: -SO₃M, -C₂H₄SO₃M, -C₃H₆SO₃M, -P(O)(OM)₂ oder -CH₂-COOM, -C₂H₄-COOM bedeuten.

### Variante C: Auf amid- oder aminhaltigen Spacern beruhende Strukturen

### C.I Geminitenside der allgemeinen Formel (C.I) analog EP 0 697 244

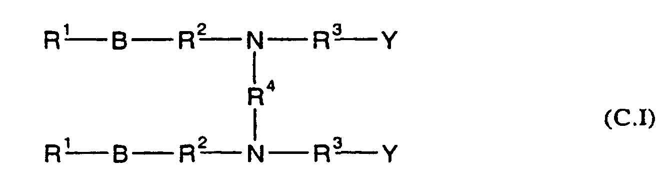

wobei die Substituenten folgende Bedeutung haben:
- R¹: C₅- bis C₂₅-Alkyl, verzweigt oder unverzweigt, gesättigt, gegebenenfalls bis zu 2-fach nicht-benachbart ungesättigt, hydroxysubstituiert oder perfluoriert ;
- R²: C₁- bis C₁₂-Alkylen oder hydroxysubstituierte Derivate davon;
- B: eine Amidgruppe [-C(O)N(R²)- oder -N(R⁵)C(O)-], eine Carboxylgruppe [-C(O)O- oder-OC(O)-], eine Polyethergruppe [-(O(R⁶-O)ₓ-];
- R⁵: für C₁- bis C₄-Alkyl oder hydroxysubstituiertes Alkyl oder H steht;
- R⁶: für C₂- bis C₄-Alkylen;
- x: eine Zahl von 1 bis 20;
- R³: für C₁- bis C₁₂- Alkyl oder hydroxysubstituierte Derivate davon, R⁷-D-R⁷ oder eine Polyethergruppe [-(O(R⁶-O)ₓ-];
- R⁷: für C₁- bis C₆- Alkylen oder hydroxysubstituierte Derivate davon;
- D: -O-, -S-, -N(R⁸)- ;
- R⁴: Alkylen oder Alkylaryl mit 1 bis 12 C-Atomen oder die hydroxysubstituierten Derivaten oder R⁹-D¹-R⁹;
- R⁸: C₁- bis C₁₂-Alkyl oder hydroxysubstituiertes Alkyl oder H oder R⁹-D¹-R⁹;
- R⁹: für C₁- bis C₆- Alkylen oder hydroxysubstituierte Derivate davon oder Aryl;
- D': -O-, -S-, -SO₂-, -C(O)-, [-(O(R⁷-O)ₓ-], (R¹⁰)₁[N(R¹⁰)]_{z} oder Aryl;
- R¹⁰: C₁- bis C₁₂-Alkyl oder hydroxysubstituiertes Alkyl oder H oder Aryl;
- t,z: unabhängig voneinander eine Zahl von 1 bis 4 bedeuten und
- Y: unabhängig voneinander für -SO₃H, O-SO₃H, -OP(O)(OH)₂, -P(O)(OH)₂, -COOH, -CO₂-C₆H₄-SO₃H und deren Salze davon steht.

### C.II Geminitenside der allgemeinen Formel (C.II) analog EP 0 697 245

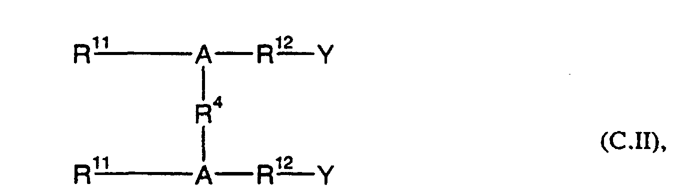

wobei die Substituenten die für die allgemeine Formel (C.I) angegebene Bedeutung haben und
- R¹¹: C₅- bis C₂₃-Alkyl, verzweigt oder unverzweigt, gesättigt, gegebenenfalls bis zu 2-fach nicht-benachbart ungesättigt, hydroxysubstituiert oder perfluoriert oder R¹⁴-B-R²;
- R¹⁴: C₁- bis C₁₂-Alkyl, verzweigt oder unverzweigt, gesättigt, gegebenenfalls bis zu 2-fach nicht-benachbart ungesättigt, oder die hydroxysubstituierten Derivate;
- R¹²: C₁- bis C₁₂-Alkylen, verzweigt oder unverzweigt, gesättigt, gegebenenfalls bis zu 2-fach nicht-benachbart ungesättigt, oder die hydroxysubstituierten Derivate oder eine Amidgruppe [-C(O)N(R²)- oder-N(R⁵)C(O)], eine Carboxylgruppe [-C(O)O- oder -OC(O)-], eine Polyethergruppe [-(O(R⁶-O)ₓ-] oder R⁹-D¹-R⁹ und
- A: -CR⁶= oder -N= unter der Voraussetzung, daß wenn A gleich -N= ist, R¹¹ gleich R¹⁴-B-R² bedeuten.

### C.III Geminitenside der allgemeinen Formel (C.III) analog DE 4227391 und DE 19608117

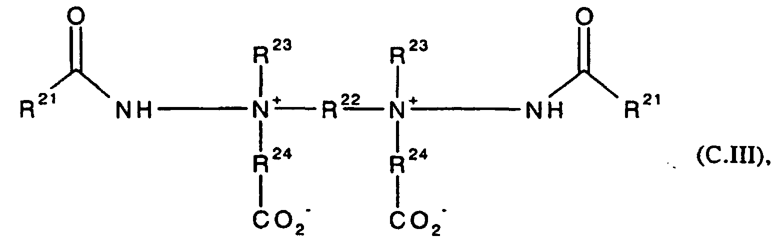

wobei die Substituenten die für die allgemeinen Formeln (C.I) und (C.II) angegebene Bedeutung haben und
- R²¹: C₅- bis C₂₃-Alkyl, verzweigt oder unverzweigt, gesättigt, gegebenenfalls bis zu 2-fach nicht-benachbart ungesättigt;
- R²², R²⁴: C₁- bis C₆ Alkylen;
- R²³: Methyl, Ethyl, Propyl oder eine Polyethergruppe [-(O(R⁶-O)ₓ-]
bedeuten.

### Variante D :

### D. I Geminitenside der allgemeinen Formel (D.I) analog US 5,863,886

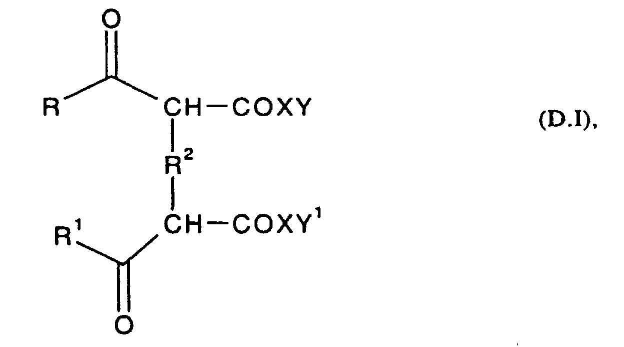

wobei die Substituenten folgende Bedeutung haben:
- R, R¹: C₅- bis C₃₀-Alkyl, verzweigt oder unverzweigt, gesättigt, gegebenenfalls bis zu 2-fach nicht-benachbart ungesättigt, hydroxysubstituiert oder perfluoriert;
- R²: C₁-bis C₁₀- Alkylen, Arylen und hydroxysubstituierte Derivat, ein Polyether [-O(R⁴O)ₓ-], -S-, -SO₂-, -O-, -S-S-, -O-R⁵-O- oder -S-R⁵-S-; Variable für eine direkte Bindung zwischen den beiden α-Kohlenstoffen;
- R⁴: C₂- bis C₄-Alkylen;
- R⁵: C₁- bis C₁₀-Alkylen, Arylen oder Alkylarylen, -N(R⁶)- oder -(NR⁶)-R⁷-(NR⁶)-
- R⁶: C₁- bis C₆-Alkyl;
- R⁷: C₁- bis C₆-Alkyl, wobei R⁷ und R⁶ auch Teil eines heterocyclischen Ringes sein können;
- X: Polyether [-O(R⁴O)ₓ-], wobei x eine Zahl von 1 bis 30 ist, -O-, NZ;
- Z: C₁- bis C₁₀- Alkyl, Aryl, Alkylaryl oder H und
- Y, Y¹: unabhängig voneinander H, -CH₂-COOH und Salze, ein Kohlenwasserstoffrest mit mindestens 2 Hydroxylgruppen, wie Erythrose, Threose, Ribose, Arabinose, Xylose, Fructose, Lyxose, Allose, Altrose, Glucose, Mannose, Galactose und ihre Mischungen.

### D.II Geminitenside der allgemeinen Formel (D.II)

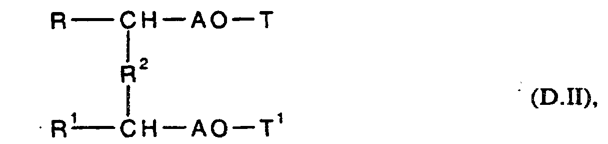

wobei die Substituenten die für die allgemeine Formel (D.I) angegebene Bedeutung haben und
- AO: -C(O)-, -C(O)- [-O(R⁴O)ₓ-], -CH₂- [-O(R⁴O)ₓ-], -CH₂-O-;
- T, T': unabhängig voneinander -OM, -H, -CH₃, -C₂H₅, -SO₃M, -CH₂COOM, -C₂H₄-COOM, -C₃H₆-SO₃M, -O-P(O)(OM)₂ und
- M: Alkyli, ½ Erdalkali, Ammonium, Mono-, Di-, Trialkanolammonium oder H bedeuten.

### D.III Geminitenside der allgemeinen Formel (D.III) analog WO 96/16930

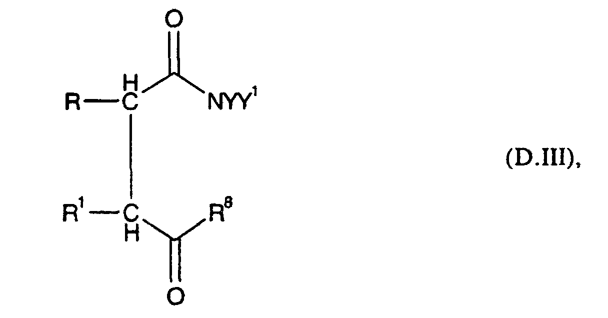

wobei die Substituenten die für die allgemeinen Formeln (D.I) und (D.II) angegebene Bedeutung haben und
- R⁸: NYY¹, -O(R⁴O)ₓH oder -O(R⁴O)ₓ-C(O)-CHR-CHR¹-C(O)NYY¹ bedeutet.

### D.IV Geminitenside der allgemeinen Formel (D.IV) analog WO 96/25384

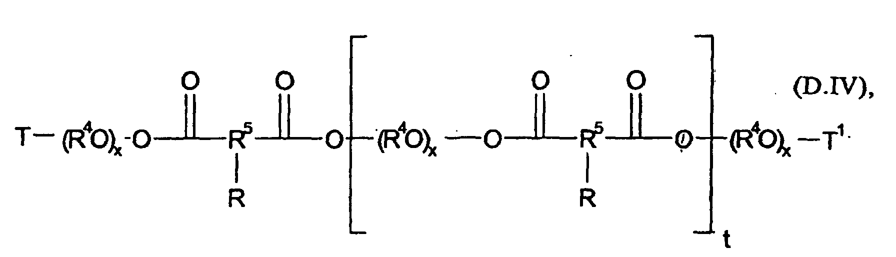

wobei die Substituenten die für die allgemeinen Formeln (D.I), (D.II) und (D.III) angegebene Bedeutung haben und
- t: eine ganze Zahl von 1 bis 100, bevorzugt 1 bis 20, besonders bevorzugt 1 bis 4 bedeutet.

Vorzugsweise werden in den Tensidzusammensetzungen als Detergens - Komponente mit milder, schlecht-schäumender Charakteristik Verbindungen **ge**mäß den Anprüche 5 bis 8 eingesetzt.

Besondere Erwähnung als Detergens - Komponente sollen bezüglich der Acyllactylate Salze, insbesondere das Natriumsalz, des Lauroyllactylates oder des Steraoyllactylates finden.

Mild im Sinne der Erfindung bedeutet, daß die entsprechenden Verbindungen / Zusammensetzung kennzeichnungsfrei (z.B. gemäß Richtlinie 67-548-EWG, Gefahrstoffverordnung) hinsichtlich ihres Irritationspotentials für Haut und Augen sind.

Unter der Eigenschaft "mit schlecht-schäumender Charakteristik" ist im Sinne der Erfindung zu verstehen, daß diese Tenside in ihrer Eigenschaft als weitere Detergens-Komponente zwei von drei der unten aufgeführten Werte bei der Beurteilung ihres Schaumbildungsverhaltens unterschreiten.

Die Kriterien sind die
- Schaumlamellendicke in mm direkt nach der Schaumherstellung,
- der Blasenzahl pro Bildfläche bei 100facher Vergrößerung direkt nach der Herstellung, beide durch die Mikroskopie des Schaumes bestimmt, und
- das Anschäumverhalten im Handversuch.

Die Ölkomponente besteht aus oder enthält vorzugsweise Pflanzenöle und Esteröle, d.h. z.B. Triglyceride von C₄- bis C₂₆ - Fettsäuren, verzweigt, unverzweigt, ein bis dreifach ungesättigt, oder Paraffinöle, d.h. Kohlenwasserstoffe bis zur Kettenlängen C₁₆ (flüssig, darüber hinaus fest) oder Siliconöle. Typische Beispiele für Pflanzenöle sind : Sonnenblumen-, Raps-, Sojaöl, Lavendel-, Anis-, Rosmarin-, Fichtennadel- und Latschenkiefemöl, Teebaumöl, Calendula-Öl, Nachtkerzenöl oder Pflegeöle, wie Avocado-, Jojobaöl oder Aloe Vera.

### Versuchsdurchführung

8 Gew.-% des zu charakterisierenden Tensids werden in demineralisiertem Wasser gelöst. Die zu untersuchende Tensidlösung wird 10 min. bei 1500 U/min mit dem Blattrührer gerührt, wobei sich die Lösung etwas erwärmt (von Raumtemperatur auf ca. 35°C). Nach 10 Minuten Rührzeit wird der entstandene Schaum von oben abgeschöpft und direkt mikroskopiert - hierbei wird die Lamellendicke in mm gemessen und die Anzahl der Schaumblasen im Bildausschnitt bestimmt.

Neben der durch Rühren erzeugten Schaumqualität wird auch das Anschäumverhalten eines Tensids unter fließendem kalten Leitungswasser beurteilt. Hierzu werden 2 g Tensid auf die Innenhandflächen verteilt und unter fließendem Wasser gleichmäßig verrieben. Die Schaumqualität wird in vier Stufen beurteilt, 0 = keine Schaumbildung, 1 = mäßige Schaumbildung, 2 = gute Schaumbildung und 3 = sehr gute Schaumbildung.

Als schlecht schäumend werden Tenside beurteilt, wenn sie die Grenzen von mindestens zwei von drei Kriterien unterschreiten, d.h. Lamellendicken kleiner oder gleich 16 mm aufweisen oder kleiner oder gleich 16 Blasen im Bildausschnitt aufweisen oder im Anschäumverhalten mit 1 oder schlechter beurteilt werden.

Als gut schäumend werden Tenside beurteilt, wenn sie alle drei Kriterien mit Werten von ≥ 20 mm in der Lamellendicke, ≥ 20 Blasen im Bildausschnitt (beides direkt nach dem Anschäumen) und ein mit 3 beurteiltes Anschäumverhalten aufweisen.

Die erfindungsgemäßen Mischungen können in Formulierungen für die Haar- und Hautreinigung eingesetzt werden als Shampoos, Babyshampoos, Antischuppenshampoos, Aerosolshampoos, Aerosolduschgele, Wasch-, Dusch- und Badegele, Schaumbäder, Ölschaumbäder, Gesichtswaschcremes, flüssige Handwaschseifen, Syndetseifen und Stückseifen des Kombibar-Typs. Hierbei werden sie entweder allein als Detergens-Bestandteil eingesetzt oder als Additiv in Kombination mit anderen Detergentien. Geeignet sind 0,1 bis 90 Gew.-%, bevorzugt 1 bis 30 Gew.-% und besonders bevorzugt 1 bis 15 Gew.-% der erfindungsgemäßen Mischung in den beschriebenen Formulierungen.

Von den sonstigen Ingredientien, mit denen die erfindungsgemäßen Mischungen bei der Herstellung von Formulierungen für die Haar- und Hautreinigung kombiniert werden können, seien beispielsweise genannt: Alkylsarcosinate, Fettalkoholethersulfate, Fettalkoholsulfate, Imidazolinderivate, Taurate, Sulfobetaine, Olefinsulfonate, Ethercarbonsäuren und deren Salze, Alkylaminoxide, ethoxylierte Fettalkohole, ethoxylierte Fettsäuren und deren Diester, Ester aus ethoxylierten Polyolen, Fettsäure-di- und -monoethanolamide, Fettsäure-mono-, -di- ,und -triglyceride sowie deren Derivate (-sulfate, -lactylate, -lactate, -citrate, -tartrate), ethoxylierte Rhizinusöle und gehärtete Rhizinusölderivate, Phospholipide, kationische Tenside und Polymere, Antischuppenmittel, Stärkederivate, Glycerinester und deren Ethoxylate, Polyglycerinester, Sorbitanester und deren Ethoxylate, Siliconöle, Siliconcopolymere, Panthenol, Panthenolether, Vitamin E und deren Dreivate, Vitamin A und deren Derivate, Zitronensäure, Milchsäure, Hyaluronsäure, Polyvinylpyrrolidon, Polyacrylate, Xanthan Gummi, Proteinhydrolysate, Acylglutamate. UVB-Filter (öllöslich oder wasserlöslich); als öllösliche Substanzen sind z. B. zu nennen: 3-Benzylidencampher und dessen Derivate wie 344-(Methylbenzyliden)campher, 4-Aminobenzoesäure-Derivate, vorzugsweise 4-Dimethylamino-benzoesäure(2- e-thylhexyl)ester, (4-Dimethylamino)-benzoe-säureamylester, Ester der Zimtsäure, vorzugsweise 4-Methoxyzimtsäure(2-ethylhexyl)ester, 4-Methoxyzimtsäureisopentylester; Ester der Salicylsäure, vorzugsweise Salicylsäure-(2-ethylhexyl)ester, Salicylsäure-(4-isopropylbenzyl)ester, Salicylsäurehomomenthylester; Derivate des Benzophenons, vorzugsweise 2-Hydroxy-4-methoxybenzophenon, 2-Hydroxy-4-methoxy-methylbenzophenon, - 2,2'-Dihydroxy-4-methoxybenzophenon, Ester der Benzylmalonsäure. Als wasserlösliche Substanzen sind vorteilhaft; 2-Phenylbenzimidazol-5-sulfonsäure und deren Salze, z. B. Natrium-, Kalium- oder Triethanolammonium-Salze, Sulfonsäure-Derivate von Benzophenonen und ihre Salze; Sulfonsäure-Derivate des 3-Benzylidencamphers und ihre Salze.

In den nachfolgenden Beispielen wird die Erfindung zusätzlich beschrieben.

### Beispiele

| Verwendete Geminitenside : | |
|---|---|
| Geminitensid (allgemeine Formel) | Struktur |
| A.A | R¹ = R³ = C₁₁H₂₃/C₁₃H₂₇, R² = C₂H₄, |
| (A.I) | X = Y = (C₂H₄O-)ₓ(C₃H₆O-)_{y}SO₃Na mit x = 14, y = 0 |
| B.A | R¹ = R³ = C₁₁ H₂₃/C₁₃H₂₇, R²= C₂H₄, A = CH₂, M = Na |
| (B.I) | |
| B.B | R⁵ = R⁶ = C₁₂H₂₅/C₁₄H₂₉, X = C₂H₄, Y¹ = CO₂Na, |
| (B.II) | Y² = -O-C(O)-C₂H₄-CO₂Na |
| B.C | R⁵ = R⁶ = C₁₂H₂₅/C₁₄H₂₉, X = C₂H₄, Y¹ = CO₂Na, Y² = -O-C(O)-C₂H₄-CO₂Na |
| (B.II) | |
| C.A | R¹ = C₁₁H₂₃, B = C₂H₄, R³ = CH₂, R⁴ = C₂H₄, |
| (C.I) | Y = COONa |
| D.A | R, R¹ = -C₁₁H₂₃, R² = -S-, X = NZ, Z = -CH₃, Y, |
| (D.I) | Y¹ = Glucosylrest |
| D.B | R, R¹ = -C₁₁H₂₃, R² = Einfachbindung, |
| (D.II) | AO = -C(O)-, T, T' 1 = OM, M = Na |
| D.C | R, R¹ = C₁₂H₂₄, R⁸ = NYY1, Y = -CH₃, |
| (D.III) | Y¹ = Glucosylrest |

### Untersuchungsmethoden

Zur Charakterisierung der Schäume wurden jeweils 8 Gew.-% der Tenside und Tensidmischungen in demineralisiertem Wasser gelöst. Die zu untersuchenden Tensidlösungen wurden jeweils 10 min. bei 1500 U/min mit dem Blattrührer gerührt, wobei sich die Lösung etwas erwärmt (von Raumtemperatur auf ca. 35°C).

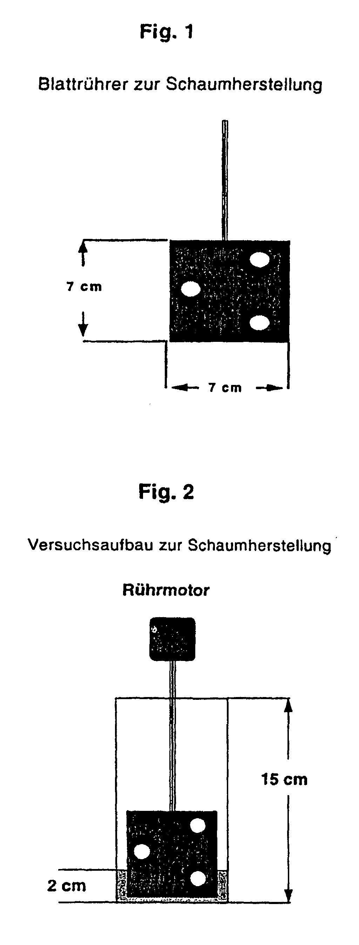

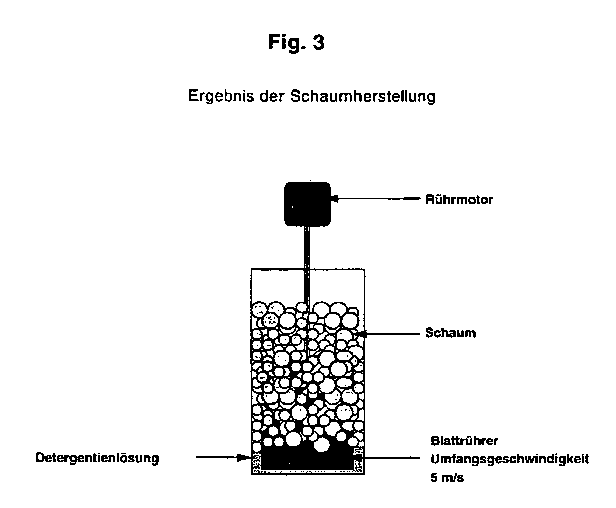

Das verwendete Verfahren zur Schaumherstellung ist in Fig. 1 bis 3 schematisch dargestellt. Die Zeichnung zeigt schematisch in Fig. 1 den verwendeten Blattrührer zur Schaumherstellung, in Fig. 2 den Versuchsaufbau zur Schaumherstellung - jeweils mit den Größenangaben in cm, wobei H die Höhe der ungeschäumten Lösung ist -, und in Fig. 3 den Zustand nach Aufschäumen bei einer Umfangsgeschwindigkeit des Blattrührers von 5 m/sec. (S = Schaum, D = Detergenslösung).

Nach 10 Minuten Rührzeit wurde der entstandene Schaum von oben abgeschöpft und sowohl direkt als auch nach 2, 5 und 15 Minuten mikroskopiert. Für alle Untersuchungen wurde folgender Versuchsaufbau gewählt, um den Einfluß der Tensidmischungen auf das Schaumbildungsverhalten zu evaluieren:

| | **(1)** | **(2)** | **(3)** | **(4)** | **(5)** | **(6)** |
|---|---|---|---|---|---|---|
| | [%w/w] | [%w/w] | [%w/w] | [%w/w) | [%w/w] | [%w/w] |
| **Phase A** | | | | | | |
| Tensid | 8,0 | 6,4 | 4,8 | 3,2 | 1,6 | |
| **Geminitensid A.A** | | 1,6 | 3,2 | 4,8 | 6,4 | 8,0 |
| **Phase B** | | | | | | |
| Demin. Wasser | 92,0 | 92,0 | 92,0 | 92,0 | 92,0 | 92,0 |
| **Summe** | **100,0** | **100,0** | **100,0** | **100,0** | **100,0** | **100,0** |

### Herstellung:

- Phase A einwiegen und bei 80°C zusammenschmelzen
- Erkalten lassen und Phase B dazugeben
- 10 min mit dem Blattrührer verrühren
- Probennahme 0, 2, 5 und 15 Minuten nach Herstellung

Für die Beurteilung der Schaumqualität wurde die Lamellendicke und die Anzahl der zu beobachtenden Blasen pro Bildfläche bei 100 facher Vergrößerung herangezogen. Es ist bekannt, daß mit zunehmender Lammellendicke mehr Reinigungsflüssigkeit über das Vehikel Schaum zur Benetzung der Haut und zur Schmutzaufnahme zur Verfügung steht, d.h. je dicker die erzeugte Schaumlamelle je besser ist die zu erwartende Reinigungswirkung. Die Anzahl der Blasen pro Fläche spiegelt die Feinporigkeit des Schaumes wider und kann als Kriterium für das Hautgefühl angesehen werden.

Nachfolgend sind die Ergebnisse der Schaumanalyse mit Bezug auf die Schaumlamellendicke (ausgedrückt in mm) bei 100 facher Vergrößerung für Mischungen von einem Geminitensid des Typs Natriumdiamidethersulfat mit verschiedenen milden und für die Haar- und Hautreinigung unzureichend schäumenden Tensiden aufgeführt.

### Beispiel 1

Schaumlamellendicke in Abhängigkeit des Mischungsverhältnisses zwischen Natriumlauroyllactylate und Geminitensid.

| | **(1)** | **(2)** | **(3)** | **(4)** | **(5)** | **(6)** |
|---|---|---|---|---|---|---|
| Zeit [min] | 8,0/0,0 | 6,4/1,6 | 4,8/3,2 | 3,2/4,8 | 1,6/6,4 | 0,0/8,0 |
| 0 | 4 | 12 | 22 | 28 | 18 | 3 |
| 2 | 2 | 10 | 22 | 25 | 15 | 2 |
| 5 | 1 | 8 | 18 | 22 | 14 | <1 |
| 15 | <1 | 6 | 12 | 20 | 13 | <1 |

### Beispiel 2

Anzahl der Schaumblasen in Abhängigkeit des Mischungsverhältnisses zwischen Natriumlauryllactylate und Geminitensid.

| | **(1)** | **(2)** | **(3)** | **(4)** | **(5)** | **(6)** |
|---|---|---|---|---|---|---|
| Zeit [min] | 8,0/0,0 | 6,4/1,6 | 4,8/3,2 | 3,2/4,8 | 1,6/6,4 | 0,0/8,0 |
| 0 | 4 | 12 | 22 | 28 | 18 | 3 |
| 2 | 2 | 10 | 22 | 25 | 15 | 2 |
| 5 | 1 | 8 | 18 | 22 | 14 | <1 |
| 15 | <1 | 6 | 12 | 20 | 13 | <1 |

### Beispiel 3

Anschäumverhalten von Natriumdiamidethersulfaten mit verschiedenen milden wenig schäumenden sehr milden Tensiden beim Verreiben mit den Händen unter fließendem kalten Wasser.

| | **(1)** | **(2)** | **(3)** | **(4)** | **(5)** | **(6)** |
|---|---|---|---|---|---|---|
| Tensid | 8,0/0,0 | 6,4/1,6 | 4,8/3,2 | 3,2/4,8 | 1,6/6,4 | 0,0/8,0 |
| Natriumlauryllactylate | 0 | 0 | +++ | +++ | + | 0 |

| Tenside | 8,0/0,0 | 6,4/1,6 | 4,8/3,2 | 3,2/4,8 | 1,6/6,4 | 0,0/8,0 |
|---|---|---|---|---|---|---|
| Natriumlauryllactylate und Gemini B.A | 0 | 0 | +++ | +++ | + | 0 |

| Tenside | 8,0/0,0 | 6,4/1,6 | 4,8/3,2 | 3,2/4,8 | 1,6/6,4 | 0,0/8,0 |
|---|---|---|---|---|---|---|
| Natriumlauryllactylate und Gemini D.A | 0 | 0 | +++ | +++ | + | 0 |
| Schlüssel für die Beurteilung der Schaumqualität | | | | | | |
| 0 = keine Schaumbildung + = mäßige Schaumbildung | | | | | | |
| ++ = gute Schaumbildung +++ = sehr gute Schaumbildung | | | | | | |

### Beispiel 4 (Formulierungsbeispiele, Mildes Shampoo)

| **Handelsname** | **CTFA/INCI** | **Modell-Formul. *** | **Vergleich*** | **Form. * A** | **Form. * B** |
|---|---|---|---|---|---|
| Texapon NSO | Sodium laureth sulfate | 40,00 | 40,00 | 40,00 | 40,00 |
| Tensidzusammensetzung | Gemini A.I, Sodium lauroyl lactylate (50 : 50) | 8,0 | 0 | 4,00 | 8,00 |
| Tego Betain F 50 | Cocoamidopropyl betaine | 0,0 | 8,00 | 0,00 | 0,00 |
| | | | | | |
| D-Panthenol 75L | Panthenol | 1,5 | 0,00 | 0,00 | 0,00 |
| Octopyrox | | 3,0 | 0,00 | 0,00 | 0,00 |
| Ucare polymer JR-400 | Polyquaternium-10 | 0,15 | 0,00 | 0,00 | 0,00 |
| Antil 141 liq. | Propylene glycol, PEG-55 propylene glycol oleate | 2,00 | 2,00 | 2,25 | 2,00 |
| NaCl | Sodium chloride | 1,0 | 1,00 | 1,25 | 1,00 |
| Zitronensäure | Citric acid | 0,75 | 0,00 | 0,00 | 0,00 |
| demin. Wasser | Aqua | ad 100 | ad 100 | ad 100 | ad 100 |

| | | | | | |
|---|---|---|---|---|---|
| * Alle Angaben in Gew.% | | | | | |

### Produktion : Bei 40 °C mischen, anschließend mit Antil 141 liq. und NaCl verdikken

Um die Hautverträglichkeit der erfindungsgemäßen Tensidzusammensetzung zu untersuchen, wurde das nur Tensidsystem einer typischen Shampoo-Formulierung (Alkoholethersulfat + milderndes Additiv + Verdickungssystem) zusammen mit der erfindungsgemäßen Tensidzusammensetzung eingesetzt.

Dieses System wurde einem Repetitiven Epikutantest nach Shelanski (mit 20 Probanden) unterzogen:

Mit dem jeweiligen Testprodukt (2%ig in Wasser) gefüllte Kunststoffkammern wurden für jeweils 24 h unter Okklusion neunmal im Zeitraum von drei Wochen an der gleichen Hautstelle befestigt. Nach 2 Wochen wurde durch erneute Applikation des Produktes in Kunststoffkammern für 24 h an derselben Stelle und an einer nicht exponierten Stelle auf eine möglicherweise induzierte Hautreaktion geprüft. Die Ablesung erfolgte unmittelbar nach Entfernen der Testkammern sowie nach 24 h, 48 h und 72 h.

Aufgrund der Testergebnisse können die Formulierungen A und B als "sehr gut hautverträglich" bezeichnet werden. Es gibt keine Anzeichen auf Hautsensibilisierung. Hinsichtlich einer eventuell hautreizenden Wirkung ist es als unbedenklich einzustufen.

Bei der heute typischen Kombination Alkoholethersulfat und Betain, der Vergleichsformulierung, konnten im Rahmen des Testes bei drei Testpersonen leichte Hautunverträglichkeiten (Rötungen, Schuppungen) beobachtet werden.

### Beispiel 5 (Formulierungsbeispiele, Mildes Duschgel

| **Handelsname** | **CTFA/INCI Nomenklatur** | **Gew.-%** |
|---|---|---|
| Tego Betain F50 | Cocoamidopropyl betaine | 25,00 |
| Tensidzusammensetzung | Gemini A.I, Sodium lauroyl lactylate (50 : 50) | 8,00 |
| Arlatone SCI | Sodium cocoyl isothionate | 2,00 |
| Antil 141 liq. | Propylene glycol, PEG-55 propylene glycol oleate | q.s. |
| NaCl | Sodium chloride | q.s. |
| demin. Wasser | Water | ad. 100 |
| **Produktion:** | | |
| Bei 50-60°C mischen, ggf. pH-Wert mit Zitronensäure einstellen, dann verdicken. | | |

### Beispiel 6 (Formulierungsbeispiele, Antimikrobakterielle Reinigungslotion)

| **Handelsname** | **CTFA/INCI Nomenklatur** | **Gew.-%** |
|---|---|---|
| N-Cetyl-N,N,Ntrimethylammoniumbromid | Cetrimoniumbromid | 2,00 |
| Tego Betain F50 | Cocoamidopropyl betaine | 20,00 |
| DC 193 Surfactant | Dimethicone copolyol | 1,50 |
| Ucare Polymer JR 400 | Polyquaternium-10 | 0,10 |
| Tensidzusammensetzung | Gemini A.I, Sodium lauroyl lactylate (50 : 50) | 5,00 |
| Euperlan PK 3000 AM | Glycol distearate, laureth-4, cocoamidopropyl betaine | 3,00 |
| Antil 141 liq. | Propylene glycol, PEG-55 propylene glycol oleate | q.s. |
| NaCl | Sodium chloride | q.s. |
| demin. Wasser | Water | ad. 100 |
| pH nach Herstellung | | 5,1 |
| Herstellung: Bei 50-60°C mischen, dann verdicken. | | |

## Patentansprüche

1. Tensidzusammensetzung enthaltend
(A) zu 1 bis 90 Gew.%, bezogen auf die Summe der Komponenten (A) und (B), ein oder mehrere Geminitenside und
(B) zum verbleibenden Rest, bezogen auf die Summe der Komponente (A) und (B), mindestens eine weitere Detergens-Komponente mit schlecht-anschäumender Charakteristik,
wobei die Detergens-Komponente Acyllactylate und/oder Acylglutamate umfasst.

2. Tensidzusammensetzung gemäß Anspruch 1, **dadurch gekennzeichnet, daß** die Tensidzusammensetzung
(A) zu 20 bis 60 Gew.-%, bezogen auf die Komponenten (A) und (B), ein oder mehrere Geminitenside und
(B) zum verbleibenden Rest, bezogen auf die Summe der Komponente (A) und (B), ein oder mehrere Detergens-Komponenten enthält.

3. Tensidzusammensetzung gemäß einem der vorhergehenden Ansprüche enthaltend weiterhin
(C) zu mindestens 0,1 Gew.%, bezogen auf die Gesamtzusammensetzung, Wasser.

4. Tensidzusammensetzung gemäß einem der vorhergehenden Ansprüche enthaltend weiterhin
(D) zu mindestens 0,1 Gew.%, bezogen auf die Gesamtzusammensetzung, eine oder mehrere Ölkomponenten.

5. Tensidzusammensetzung gemäß einem der vorhergehenden Ansprüche, **dadurch gekennzeichnet, daß** die Detergenskomponente Acyllactylate umfasst.

6. Tensidzusammensetzung gemäß einem der vorhergehenden Ansprüche, **dadurch gekennzeichnet, daß** diese als Detergenskomponente Natrium-, Kalium-, Magnesium- oder Calciumsalze der an der Hydroxylgruppe mit linearen oder verzweigten, gesättigten oder 1 bis 3-fach nicht-benachbart ungesättigten, cyclischen oder acyclischen Carbonsäuren mit C6- bis C24- veresterten monomeren Milchsäure oder deren Oligomeren, wobei der Oligomerisierungsgrad der Milchsäure bei 1,1 bis 10, bevorzugt 1,1 bis 4 liegt, enthält.

7. Tensidzusammensetzung gemäß einem der Ansprüche 1 bis 4, **dadurch gekennzeichnet, daß** die Detergenskomponente Acylglutamate umfasst.

8. Tensidzusammensetzung gemäß einem der Ansprüche 1 bis 4, **dadurch gekennzeichnet, daß** die Acylglutamate
- Acylglutamate mit 6 bis 24 Kohlenstoffatomen in der Acylkette sind und die Acylkette linear oder verzweigt, gesättigt oder 1- bis 3-fach nicht-benachbart ungesättigt ist.

9. Tensidzusammensetzung gemäß einem der vorhergehenden Ansprüche, **dadurch gekennzeichnet, daß** das Geminitensid an der Verknüpfungsstelle zwischen Spacer, hydrophiler und hydrophober Gruppe Stickstoffatome aufweist.

10. Tensidzusammensetzung gemäß einem der vorhergehenden Ansprüche, **dadurch gekennzeichnet, daß** das Geminitensid bzw. dessen Gemisch einen Amin- oder Amidgruppen aufweisenden Spacer mit 1 bis 12 C-Atomen aufweist.

11. Tensidzusammensetzung gemäß einem der vorhergehenden Ansprüche, **dadurch gekennzeichnet, daß** das Geminitensid als hydrophobe Doppelgruppe je einen C6- bis C24-Kohlenwasserstoffrest; und/oder als hydrophile Doppel (Kopf)-gruppe mindestens einen alkoxylierten Rest aufweist, der eine Sulfonsäure-, Carbonsäure-, Phosphonsäure-, Polyalkohol- oder Polyalkylenoxid - Gruppe trägt, die gegebenenfalls auch in Salzform vorliegen kann.

12. Tensidzusammensetzung gemäß einem der vorhergehenden Ansprüche oder einem der Ansprüche 28 bis 32 **dadurch gekennzeichnet, daß** die Komponente (A) zu 1 bis 30 Gew.%, bezogen auf die Summe ionischer, vorzugsweise anionischer, Tenside, die nicht Geminitenside gemäß Komponete (A) sind, in der Tensidzusammensetzung enthalten ist.

13. Tensidzusammensetzung gemäß einem der vorhergehenden Ansprüche oder einem der Ansprüche 28 bis 31, **dadurch gekennzeichnet, daß** die Komponente (A) und (B) in der Summe zu 0,1 bis 40 Gew.%, vorzugsweise zu 0,1 bis 10 Gew.%, in der Gesamtzusammensetzung enthalten sind.

14. Tensidzusammensetzung nach einem der Ansprüche 1 bis 13, **dadurch gekennzeichnet, daß** das Geminitensid die allgemeine Formel (A.I) aufweist 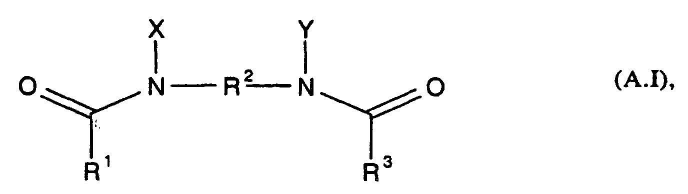 wobei die Substituenten folgende Bedeutung haben:
R¹, R³ C₅- bis C₂₅-Alkyl, verzweigt oder unverzweigt, gesättigt, gegebenenfalls bis zu 2-fach nicht-benachbart ungesättigt;
R² C₁- bis C₁₂-Alkylen;
X, Y (C₂H₄O-)ₓ(C₃H₆O-)_{y}-FR; x+y ≥ 1, x: 0-15, y: 0-10 und
FR -SO₃M, -CH₂-CO₂M, -P(O)(OM)₂, H, -C₃H₆SO₃M, -CH₂(CHOH)₄CH₂OH soweit x+y=0, wobei M = Alkali, (Alkyl)Ammonium, Alkanolammonium, H oder ½ Erdalkali.

15. Tensidzusammensetzung nach einem der Ansprüche 1 bis 13, **dadurch gekennzeichnet, daß** das Geminitensid die allgemeine Formel (A.II) aufweist 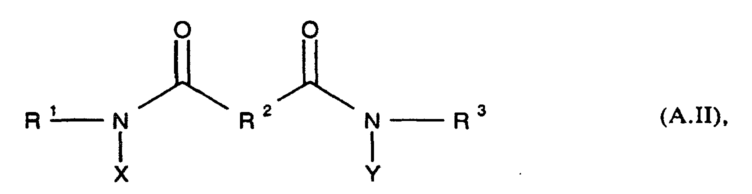 wobei die Substituenten die für die allgemeine Formel (A.I) in Anspruch 14 angegebene Bedeutung haben.

16. Tensidzusammensetzung nach einem der Ansprüche 1 bis 13, **dadurch gekennzeichnet, daß** das Geminitensid die allgemeine Formel (A.III) aufweist 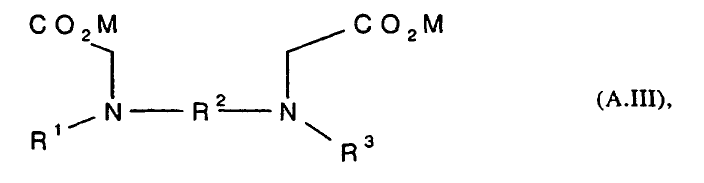 wobei die Substituenten die für die allgemeine Formel (A.I) in Anspruch 14 angegebene Bedeutung haben.

17. Tensidzusammensetzung nach einem der Ansprüche 1 bis 13, **dadurch gekennzeichnet, daß** das Geminitensid die allgemeine Formel (B.I) aufweist 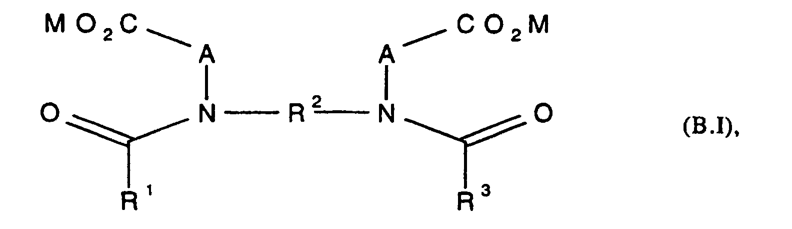 wobei die Substituenten folgende Bedeutung haben:
R¹, R³ C₅- bis C₂₅-Alkyl, verzweigt oder unverzweigt, gesättigt, gegebenenfalls bis zu 2-fach nicht-benachbart ungesättigt;
R² C₁- bis C₁₂-Alkylen;
A CHR⁴, CH₂, C₂H₄, C₃H₆, C₄H₈;
R⁴ Rest einer Aminocarbonsäure und
M Alkali, (Alkyl)Ammonium, Alkanolammonium, H oder ½ Erdalkali.

18. Tensidzusammensetzung nach einem der Ansprüche 1 bis 13, **dadurch gekennzeichnet, daß** das Geminitensid die allgemeine Formel (B.II) aufweist 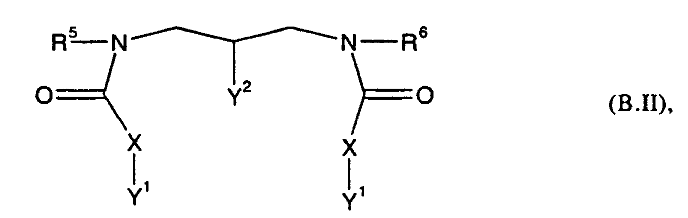 wobei die Substituenten die für die allgemeine Formel (B.I) in Anspruch 17 angegebene Bedeutung haben und
R⁵, R⁶ C6- bis C36-Alkyl, verzweigt oder unverzweigt, gesättigt, gegebenenfalls bis zu 2-fach nicht-benachbart ungesättigt;
X Alkylen- oder Alkenylengruppe mit 1 bis 6 Kohlenstoffatomen, die ggf. mit einer Hydroxylgruppe oder einer Sulfonsäuregruppe oder einer Carboxygruppe substituiert ist;
Y¹ eine Sulfonat- oder Sulfatgruppe oder eine Carboxylgrupppe und
Y² eine Hydroxylgruppe, ein Schwefelsäurerest oder -O-(CO)X-COOH bedeuten.

19. Tensidzusammensetzung nach einem der Ansprüche 1 bis 13, **dadurch gekennzeichnet, daß** das Geminitensid die allgemeine Formel (B.III) aufweist 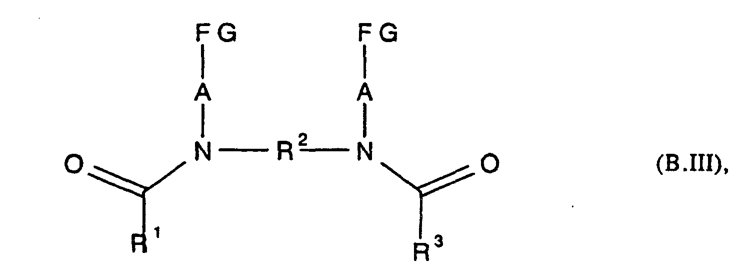 wobei die Substituenten die für die allgemeine Formel (B.I) in Anspruch 17 ange. gebene Bedeutung haben und
FG -COOM oder -SO₃M bedeutet.

20. Tensidzusammensetzung nach einem der Ansprüche 1 bis 13, **dadurch gekennzeichnet, daß** das Geminitensid die allgemeine Formel (B.IV) aufweist 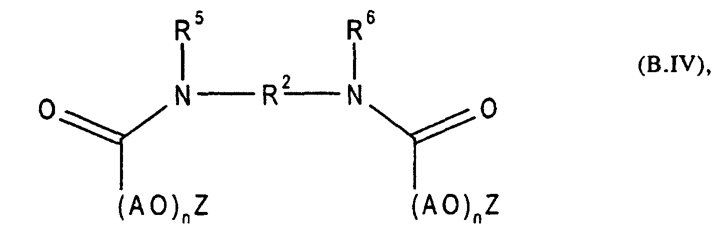 wobei die Substituenten, die bei den allgemeinen Formeln (B.I) in Anspruch 17 und (B.II) in Anspruch 18 angegebene Bedeutung haben und
AO Alkylenoxideinheiten, d.h. Ethylenglykol-, Propylenglykol und Butylenglykolethereinheiten, allein oder statistisch oder blockweise verteilt, mit n = 1 bis 20 und
Z -SO₃M, -C₂H₄SO₃M, -C₃H₆SO₃M, -P(O)(OM)₂, -CH₂-COOM oder -C₂H₄-COOM bedeuten.

21. Tensidzusammensetzung nach einem der Ansprüche 1 bis 13, **dadurch gekennzeichnet, daß** das Geminitensid die allgemeine Formel (C.I) aufweist 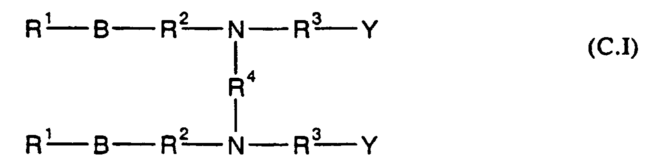 wobei die Substituenten folgende Bedeutung haben:
R¹ C₅- bis C₂₅-Alkyl, verzweigt oder unverzweigt, gesättigt, gegebenenfalls bis zu 2-fach nicht-benachbart ungesättigt, hydroxysubstituiert oder perfluoriert;
R² C₁- bis C₁₂-Alkylen oder hydroxysubstituierte Derivate davon;
B eine Amidgruppe [-C(O)N(R²)- oder -N(R⁵)C(O)-], eine Carboxylgruppe [-C(O)O- oder -OC(O)-], eine Polyethergruppe [-(O(R⁶-O)ₓ-];
R⁵ für C₁- bis C₄-Alkyl oder hydroxysubstituiertes Alkyl oder H steht;
R⁶ für C₂- bis C₄-Alkylen;
x eine Zahl von 1 bis 20;
R³ für C₁- bis C₁₂- Alkyl oder hydroxysubstituierte Derivate davon, R⁷-D-R⁷ oder eine Polyethergruppe [-(O(R⁶-O)ₓ-];
R⁷ für C₁- bis C₆- Alkylen oder hydroxysubstituierte Derivate davon;
D -O-, -S-, -N(R⁸)-;
R⁴ Alkylen oder Alkylaryl mit 1 bis 12 C-Atomen oder die hydroxysubstituierten Derivaten oder R⁹-D¹-R⁹;
R⁸ C₁- bis C₁₂-Alkyl oder hydroxysubstituiertes Alkyl oder H oder R⁹-D¹-R⁹;
R⁹ für C₁- bis C₆- Alkylen oder hydroxysubstituierte Derivate davon oder Aryl;
D' -O-, -S-, -SO₂-, -C(O)-, [-(O(R⁷-O)ₓ-], (R¹⁰)ₜ[N(R¹⁰)]_{z} oder Aryl;
R¹⁰ C₁- bis C₁₂-Alkyl oder hydroxysubstituiertes Alkyl oder H oder Aryl;
t,z unabhängig voneinander eine Zahl von 1 bis 4 bedeuten und
Y unabhängig voneinander für -SO₃H, O-SO₃H, -OP(O)(OH)₂, -P(O)(OH)₂, -COOH, -CO₂-C₆H₄-SO₃H oder deren Salze steht.

22. Tensidzusammensetzung nach einem der Ansprüche 1 bis 13, **dadurch gekennzeichnet, daß** das Geminitensid die allgemeine Formel (C.II) aufweist 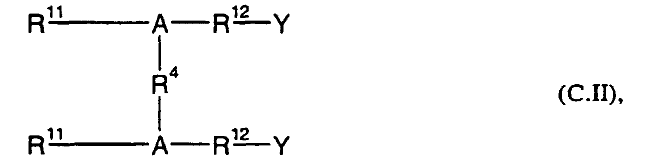 wobei die Substituenten die für die allgemeine Formel (C.I) in Anspruch 21 angegebene Bedeutung haben und
**R**^{**11**} C₅- bis C₂₃-Alkyl, verzweigt oder unverzweigt, gesättigt, gegebenenfalls bis zu 2-fach nicht-benachbart ungesättigt, hydroxysubstituiert oder perfluoriert oder R¹⁴-B-R²;
**R**^{**14**} C₁- bis C₁₂-Alkyl, verzweigt oder unverzweigt, gesättigt, gegebenenfalls bis zu 2-fach nicht-benachbart ungesättigt, oder die hydroxysubstituierten Derivate;
**R**^{**12**} C₁- bis C₁₂-Alkylen, verzweigt oder unverzweigt, gesättigt, gegebenenfalls bis zu 2-fach nicht-benachbart ungesättigt, oder die hydroxysubstituierten Derivate oder eine Amidgruppe [-C(O)N(R²)- oder -N(R-⁵)C(O)], eine Carboxylgruppe [-C(O)O- oder -OC(O)-], eine Polyethergruppe [-(O(R⁶-O)ₓ-] oder R⁹-D¹-R⁹ und
**A** -CR⁶= oder -N= unter der Voraussetzung, daß wenn A gleich -N= ist, R¹¹ gleich R¹⁴ -B-R² bedeuten.

23. Tensidzusammensetzung nach einem der Ansprüche 1 bis 13, **dadurch gekennzeichnet, daß** das Geminitensid die allgemeine Formel (C.III) aufweist 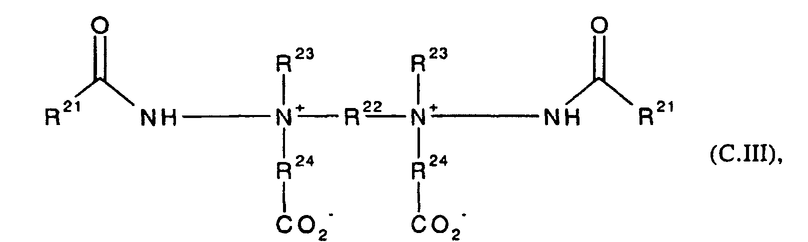 wobei die Substituenten die für die allgemeinen Formeln (C.I) und (C.II) angegebene Bedeutung haben und
R²¹ C₅- bis C₂₃-Alkyl, verzweigt oder unverzweigt, gesättigt, gegebenenfalls bis zu 2-fach nicht-benachbart ungesättigt;
R²², R²⁴ C₁- bis C₆ Alkylen und
R²³ Methyl, Ethyl, Propyl oder eine Polyethergruppe [-(O(R⁶-O)ₓ-] bedeuten.

24. Tensidzusammensetzung nach einem der Ansprüche 1 bis 13, **dadurch gekennzeichnet, daß** das Geminitensid die allgemeine Formel (D.I) aufweist 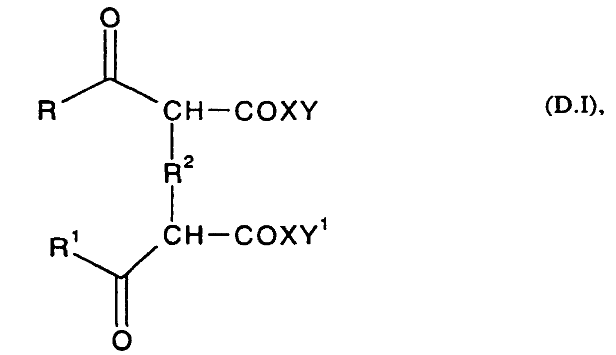 wobei die Substituenten folgende Bedeutung haben:
R, R¹ C₅- bis C₃₀-Alkyl, verzweigt oder unverzweigt, gesättigt, gegebenenfalls bis zu 2-fach nicht-benachbart ungesättigt, hydroxysubstituiert oder perfluoriert;
R² C₁-bis C₁₀- Alkylen, Arylen odere deren hydroxysubstituierte Derivate, ein Polyether [-O(R⁴O)ₓ-], -S-, -SO₂-, -O-, -S-S-, -O-R⁵-O- oder -S-R⁵S-; Variable für eine direkte Bindung zwischen den beiden α-Kohlenstoffen;
R⁴ C₂- bis C₄-Alkylen;
R⁵ C₁- bis C₁₀-Alkylen, Arylen oder Alkylarylen, -N(R⁶)- oder -(NR⁶)-R⁷-(NR⁶)- ;
R⁶ C₁- bis C₆-Alkyl;
R⁷ C₁- bis C₆-Alkyl, wobei R⁷ und R⁶ auch Teil eines heterocyclischen Ringes sein können;
X Polyether [-O(R⁴O)ₓ-], wobei x eine Zahl von 1 bis 30 ist, -O-, NZ;
Z C₁- bis C₁₀- Alkyl, Aryl, Alkylaryl oder H und
Y, Y' unabhängig voneinander H, -CH₂-COOH und Salze, Kohlenwasserstoffrest mit mindestens 2 Hydroxylgruppen, wie Erythrose, Threose, Ribose, Arabinose, Xylose, Fructose, Lyxose, Allose, Altrose, Glucose, Mannose, Galactose und ihre Mischungen.

25. Tensidzusammensetzung nach einem der Ansprüche 1 bis 13, **dadurch gekennzeichnet, daß** das Geminitensid die allgemeine Formel (D.II) aufweist 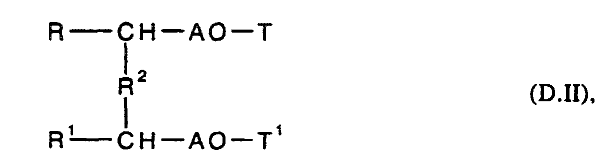 wobei die Substituenten die für die allgemeine Formel (D.I) in Anspruch 24 angegebene Bedeutung haben und
AO -C(O)-, -C(O)- [-O(R⁴O)ₓ-], -CH₂- [-O(R⁴O)ₓ-], -CH₂-O-;
T, T¹ unabhängig voneinander -OM, -H, -CH₃, -C₂H₅, -SO₃M, -CH₂COOM, -C₂H₄-COOM, -C₃H₆-SO₃M, -O-P(O)(OM)₂ und
M Alkyli, ½ Erdalkali, Ammonium, Mono-, Di-, Trialkanolammonium oder H bedeuten.

26. Tensidzusammensetzung nach einem der Ansprüche 1 bis 13, **dadurch gekennzeichnet, daß** das Geminitensid die allgemeine Formel (D.III) aufweist 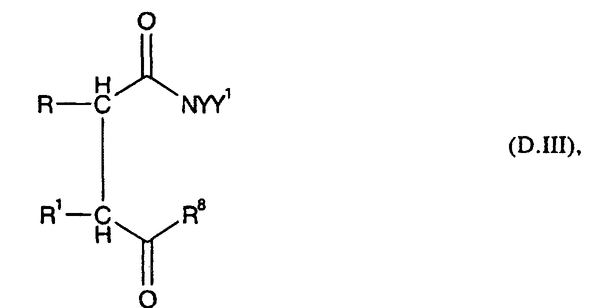 wobei die Substituenten die für die allgemeinen Formeln (D.I) in Anspruch 24 und (D.II) in Anspruch 25 angegebene Bedeutung haben und
R⁸ NYY¹, -O(R⁴O)ₓH oder -O(R⁴O)ₓ-C(O)-CHR-CHR¹-C(O)NYY¹ bedeutet.

27. Tensidzusammensetzung nach einem der Ansprüche 1 bis 13, **dadurch gekennzeichnet, daß** das Geminitensid die allgemeine Formel (D.IV) aufweist 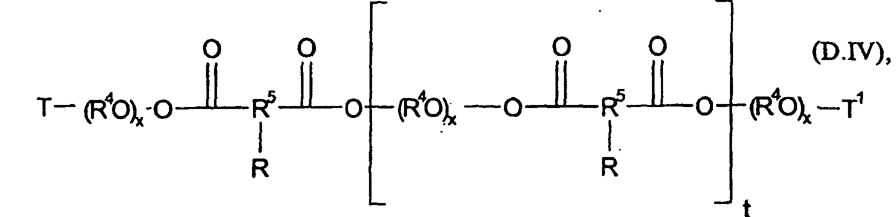 wobei die Substituenten die für die allgemeinen Formeln (D.I) in Anspruch 24, (D.II) in Anspruch 25 und (D.III) in Anspruch 26 angegebene Bedeutung haben und
t eine ganze Zahl von 1 bis 100, bevorzugt 1 bis 20, besonders bevorzugt 1 bis 4 bedeutet.

28. Tensidzusammensetzung gemäß einem der Ansprüche 1 bis 4, **dadurch gekennzeichnet, daß** die Tensidzusammensetzung als Komponente (A) ein Geminitensid der allgemeinen Formel (AI) und als Komponente (B) Acyllactylat, und/oder Acylglutamate enthält.

29. Tensidzusammensetzung gemäß einem der Ansprüche 1 bis 4, **dadurch gekennzeichnet, daß** die Tensidzusammensetzung als Komponente (A) ein Geminitensid der allgemeinen Formel (AIII) und als Komponente (B) Acyllactylate und/oder Acylglutamate enthält.

30. Tensidzusammensetzung gemäß einem der Ansprüche 1 bis 4, **dadurch gekennzeichnet, daß** die Tensidzusammensetzung als Komponente (A) ein Geminitensid der allgemeinen Formel (CII) und als Komponente (B) Acyllactylate und/oder Acylglutamate enthält.

31. Tensidzusammensetzung gemäß einem der Ansprüche 1 bis 4, **dadurch gekennzeichnet, daß** die Tensidzusammensetzung als Komponente (A) ein Geminitensid der allgemeinen Formel (DI) und als Komponente (B) Acyllactylate und/oder Acylglutamate enthält.

32. Verwendung der Tensidzusammensetzung gemäß einem der vorhergehenden Ansprüche als Inhaltsstoff von Haar- und Hautreinigungsmittel-Formulierungen.

## Claims

1. A surfactant composition comprising
(A) 1 to 90 wt%, referring to the total amount of components (A) and (B), of one or more gemini surfactant(s) and,
(B) referring to the remainder, based on the total of components (A) and (B), at least one additional detergent component with poor inital foaming characteristics, wherein the detergent component comprises acyllactylates and/or acylglutamates.

2. The surfactant composition of Claim 1, **characterized in that** the surfactant composition comprises
(A) 20 to 60 wt%, referring to the components (A) and (B), of one or more gemini surfactant(s) and,
(B) referring to the remainder, based on the total of components (A) and (B), one or more detergent component(s).

3. A surfactant composition according to any one of the preceding claims further comprising
(C) at least 0.1 wt% water, referring to the total composition.

4. A surfactant composition according to any one of the preceding claims further comprising
(D) at least 0.1 wt% of one or more oil component(s), referring to the total compositon.

5. A surfactant composition according to any one of the preceding claims, **characterized in that** the detergent component comprises acyllactylates.

6. A surfactant composition according to any one of the preceding claims, **characterized in that** it comprises as a detergent component sodium-, potassium-, magnesium-, or calcium salts of at the hydroxyl group of linear or branched, saturated or non-adjacently mono- to triunsaturated, cyclic or acyclic carboxylic acids with C₆ to C₂₄ esterified monomeric lactic acid, or the oligomers thereof, the oligomerization degree of the lactic acid being in the range of from 1.1 to 10, preferably 1.1 to 4.

7. A surfactant composition according to any one of claims 1 to 4, **characterized in that** the detergent component comprises acylglutamates.

8. A surfactant composition according to any one of claims 1 to 4, **characterized**
**in that** the acylglutamates are
- acylglutamates having 6 to 24 carbon atoms in the acyl chain and the acyl chain is linear or branched saturated or non-adjacently mono-to triunsaturated.

9. A surfactant composition according to any one of the preceding claims, **characterized in that** the gemini surfactant comprises nitrogen atoms at the link between spacer, hydrophilic group, and hydrophobic group.

10. A surfactant composition according to any one of the preceding claims, **characterized in that** the gemini surfactant or the blend thereof comprises an amine- or amide-group-containing spacer with 1 to 12 carbon atoms.

11. A surfactant composition according to any one of the preceding claims, **characterized in that** the gemini surfactant comprises as a hydrophobic double group a C₆- to C₂₄-hydrocarbon residue each and/or as a hydrophilic double (head) group comprises at least one alkoxylated residue bearing a sulfonic acid-, carboxylic acid-, phosphonic acid -, polyalcohol-, or polyalkylene oxide group, which can also be present in salt form.

12. A surfactant composition according to any one of the preceding claims or to any one of claims 28 to 32, **characterized in that** the surfactant composition comprises 1 to 30 wt% of component (A), referring to the total amount of ionic, preferably anionic surfactants, which are no gemini surfactants in conformity with component (A).

13. A surfactant composition according to any one of the preceding claims or to any one of claims 28 to 32, **characterized in that** the components (A) and (B) are present in the whole composition in a total amount of from 0.1 to 40 wt%, preferably 0.1 to 10 wt%.

14. A surfactant composition according to any one of claims 1 to 13, **characterized**
**in that** the gemini surfactant has the general formula (A.I) 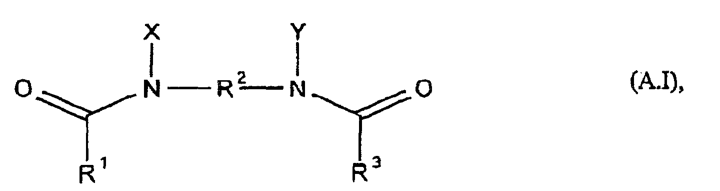 wherein the substituents have the following meanings:
R¹, R³ C₅- to C₂₅-alkyl, branched or unbranched, saturated, optionally as far as non-adjacently diunsaturated;
**R**^{**2**} C₁- to C₁₂-alkylene;
**X, Y** (C₂H₄O-)ₓ(C₃H₆O-)_{y}-FR; x+y ≥ 1, x: 0-15, y: 0-10 ; and
**FR** -SO₃M, -CH₂-CO₂M, -P(O)(OM)₂, H, -C₃H₆SO₃M, -CH₂(CHOH)₄CH₂OµH, insofar as x+y=0, wherein M = alkali, (alkyl)ammonium, alkanol ammonium, H, or ½ alkaline earth.

15. A surfactant composition according to any one of claims 1 to 13, **characterized in that** the gemini surfactant has the general formula (A.II) 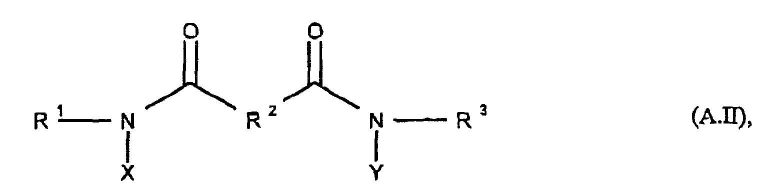 wherein the substituents have the meanings as defined by the general formula (A.I) of claim 14.

16. A surfactant composition according to any one of claims 1 to 13, **characterized in that** the gemini surfactant has the general formula (A.III) 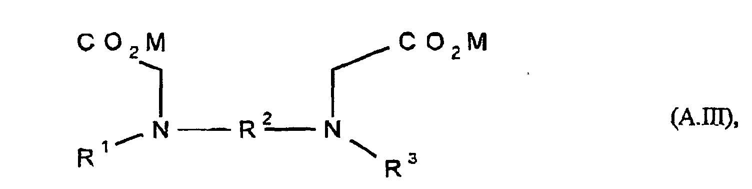 wherein the substituents have the meanings as defined by the general formula (A.I) of claim 14.

17. A surfactant composition according to any one of claims 1 to 13, **characterized in that** the gemini surfactant has the general formula (B.I) 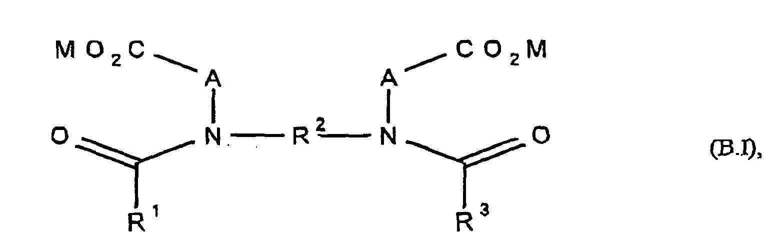 wherein the substituents have the following meanings:
**R**^{**1**}**, R**^{**3**} C₅- to C₂₅-alkyl, branched or unbranched, saturated, optionally as far as non-adjacently diunsaturated;
**R**^{**2**} C₁- to C₁₂-alkylene
**A** CHR⁴, CH₂, C₂H₄, C₃H₆, C₄H₈;
**R**^{**4**} aminocarboxylic acid radical, and
**M** alkali, (alkyl)ammonium, alkanol ammonium, H, or ½ alkaline earth.

18. A surfactant composition according to any one of claims 1 to 13, **characterized in that** the gemini surfactant has the general formula (B.II) 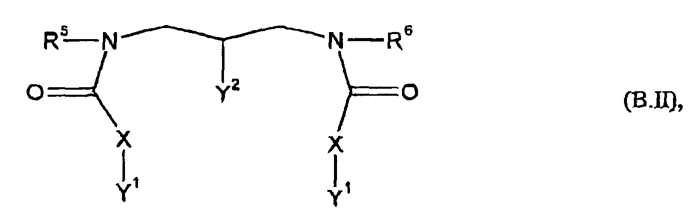 wherein the substituents have the meanings as defined by the general formula (B.I) of claim 17 and
**R**^{**5**}**, R**^{**6**} represent C₆- to C₃₆-alkyl, branched or unbranched, saturated, optionally as far as non-adjacently diunsaturated;
X is an alkylene- or alkenylene group having from 1 to 6 carbon atoms, which may be substituted with a hydroxyl group or a sulfonic acid group or a carboxy group;
Y¹ is a sulfonate- or sulfate group or a carboxyl group, and
**Y**^{**2**} represents a hydroxyl group, a sulfuric acid residue, or -O-(CO)X-COOH.

19. A surfactant composition according to any one of claims 1 to 13, **characterized in that** the gemini surfactant has the general formula (B.III) 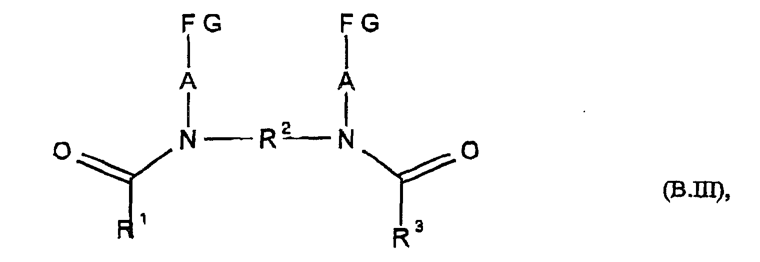 wherein the substituents have the meanings as defined by the general formula (B.I) of claim 17 and
**FG** represents -COOM or -SO₃M.

20. A surfactant composition according to any one of claims 1 to 13, **characterized in that** the gemini surfactant has the general formula (B.IV) 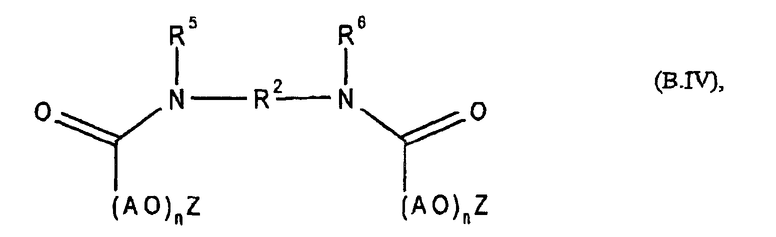 wherein the substituents have the meanings as defined by the general formulas (B.I) of claim 17 and (B.II) of claim 18 and
**AO** represents alkylene oxide units, i.e. ethyleneglycol-, propyleneglycol-, and butyleneglycol ether units, alone or arranged randomly or blockwise, wherein n = 1 to 20, and
**Z** is -SO₃M, -C₂H₄SO₃M, -C₃H₆SO₃M, -P(O)(OM)₂, -CH₂-COOM, or -C₂H₄-COOM.

21. A surfactant composition according to any one of claims 1 to 13, **characterized in that** the gemini surfactant has the general formula (C.I), 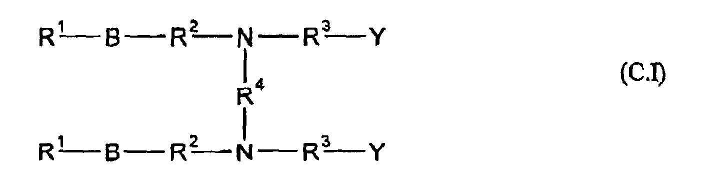 wherein the substituents have the following meanings:
**R**^{**1**} C₅- to C₂₅-alkyl, branched or unbranched, saturated, optionally as far as non-adjacently diunsaturated, hydroxy-substituted or perfluorinated;
**R**^{**2**} C₁- to C₁₂-alkylene or hydroxy-substituted derivatives thereof;
**B** an amide group [-C(O)N(R²)- or -N(R⁵)C(O)-], a carboxyl group [-C(O)O- or -OC(O)-], a polyether group [-O(R⁶-O)ₓ-];
**R**^{**5**} C₁- to C₄-alkyl or hydroxy-substituted alkyl or H;
**R**^{**6**} C₂- to C₄-alkylene;
**x** a number from 1 to 20;
**R**^{**3**} C₁- to C₁₂-alkyl or hydroxy-substituted derivatives thereof, R⁷-D-R⁷ or a polyether group [-O(R⁶-O)ₓ-];
**R**^{**7**} C₁- to C₆-alkylene or hydroxy-substituted derivatives thereof;
**D** -O-, -S-, -N(R⁸)-;
**R**^{**4**} alkylene or alkylaryl having from 1 to 12 carbon atoms or the hydroxy-substituted derivatives or R⁹-D¹-R⁹;
**R**^{**8**} C₁- to C₁₂-alkyl or hydroxy-substituted alkyl or H or R⁹-D¹-R⁹;
**R**^{**9**} C₁- to C₆-alkylene or hydroxy-substituted derivatives thereof or aryl;
**D**^{**1**} -O-, -S-, -SO₂-, -C(O)-, [-O(R⁷-O)ₓ-], (R¹⁰)ₜ[(R¹⁰)]_{z}, or aryl;
**R**^{**10**} C₁- to C₁₂-alkyl or hydroxy-substituted alkyl or H or aryl;
**t, z** are independently a number from 1 to 4, and
**Y** is independently -SO₃H, O-SO₃H, -OP(O)(OH)₂, -P(O)(OH)₂, -COOH, -CO₂-C₆H₄-SO₃H or the salts thereof.

22. A surfactant composition according to any one of claims 1 to 13, **characterized in that** the gemini surfactant has the general formula (C.II) 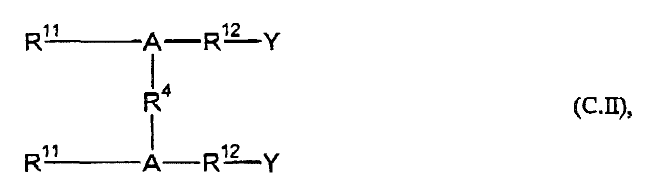 wherein the substituents have the meanings as defined by the general formula (C.I) of claim 21 and
**R**^{**11**} is C₅- to C₂₃-alkyl, branched or unbranched, saturated, optionally as far as non-adjacently diunsaturated, hydroxy-substituted or perfluorinated or R¹⁴-B-R²;
**R**^{**14**} is C₁- to C₁₂-alkyl, branched or unbranched, saturated, optionally as far as non-adjacently diunsaturated, or the hydroxy-substituted derivatives;
**R**^{**12**} means C₁- to C₁₂-alkylene, branched or unbranched, saturated, optionally as far as non-adjacently diunsaturated, or the hydroxy-substituted derivatives, or an amide group [-C(O)N(R²)- or -N(R⁵)C(O)-], a carboxyl group [-C(O)O- or -OC(O)-], a polyether group [-O(R⁶-O)ₓ-] or R⁹-D¹-R⁹ and
**A** is -CR⁶= or -N=, if whenever A is equal to -N=, R¹¹ represents R¹⁴-B-R².

23. A surfactant composition according to any one of claims 1 to 13, **characterized in that** the gemini surfactant has the general formula (C.III) 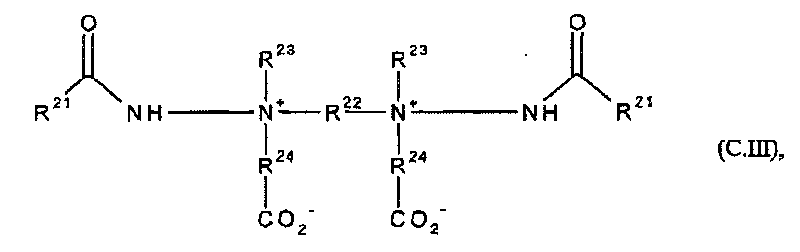 wherein the substituents have the meanings as defined by the general formulas (C.I) and (C.II) and
**R**^{**21**} represents C₅- to C₂₃-alkyl, branched or unbranched, saturated, optionally as far as non-adjacently diunsaturated;
**R**^{**22**}**, R**^{**24**} are C₁- to C₆-alkylene and
**R**^{**23**} is methyl, ethyl, propyl, or a polyether group [-O(R⁶-O)ₓ-].

24. A surfactant composition according to any one of claims 1 to 13, **characterized in that** the gemini surfactant has the general formula (D.I) 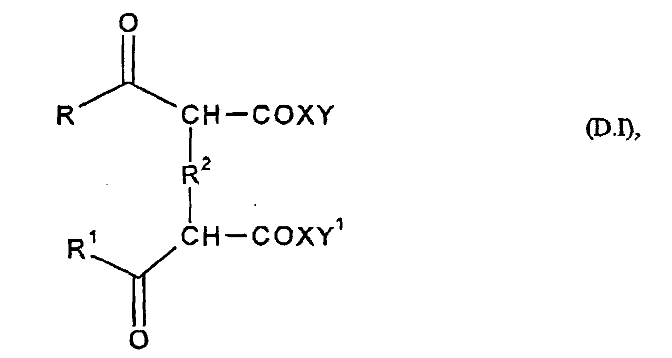 wherein the substituents have the following meanings:
**R, R**^{**1**} C₅- to C₃₀-alkyl, branched or unbranched, saturated, optionally as far as non-adjacently diunsaturated, hydroxy-substituted or perfluorinated;
**R**^{**2**} C₁- to C₁₀-alkylene, arylene, or the hydroxy-substituted derivatives thereof, a polyether [-O(R⁴O)ₓ-], -S-, -SO₂- -O-, -S-S-, -O-R⁵-O-, or -S-R⁵-S-; variable for a direct bond between the two α-carbons;
**R**^{**4**} C₂- to C₄-alkylene;
**R**^{**5**} C₁- to C₁₀-alkylene, arylene, or alkyl arylene, -N(R⁶)-, or -(NR⁶)-R⁷-(NR⁶)-;
**R**^{**6**} C₁- to C₆-alkyl;
**R**^{**7**} C₁- to C₆-alkyl, wherein R⁷ and R⁶ can also be part of a heterocyclic ring;
**X** polyether [-O(R⁴O)ₓ-], wherein x is a number from 1 to 30, -O-, NZ;
**Z** C₁- to C₁₀-alkyl, aryl, alkylaryl, or H, and
**Y, Y**^{**1**} are independently H, -CH₂-COOH and salts, a hydrocarbon radical having at least two hydroxyl groups, such as erythrose, threose, ribose, arabinose, xylose, fructose, lyxose, allose, altrose, glucose, mannose, galactose, and mixtures thereof.

25. A surfactant composition according to any one of claims 1 to 13, **characterized In that** the gemini surfactant has the general formula (D.II) 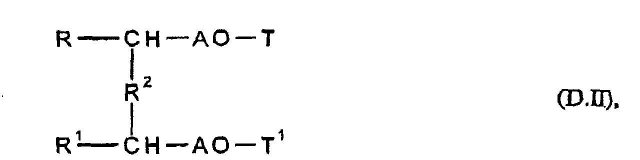 wherein the substituents have the meanings as
defined by the general formula (D-I) of claim 24 and
**AO** means -C(O)-, -C(O)- [-O(R⁴O)ₓ-], -CH₂-[-O(R⁴O)ₓ-], -CH₂-O-;
**T, T**^{**1**} are independently -OM, -H, -CH₃, -C₂H₅, -SO₃M, -CH₂COOM, -C₂H₄-COOM, -C₃H₆-SO₃M, -O-P(O)(OM)₂ and
**M** is alkyli, ½ alkaline earth, ammonium, mono-, di-, trialkanolammonium, or H.

26. A surfactant composition according to any one of claims 1 to 13, **characterized in that** the gemini surfactant has the general formula (D.III) 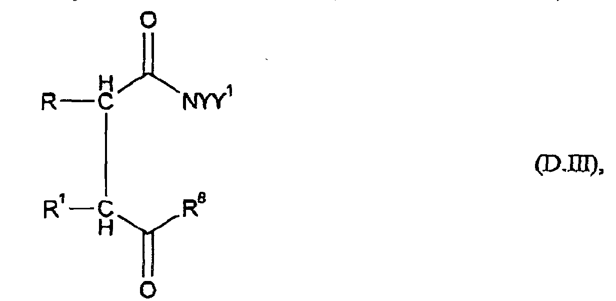 wherein the substituents have the meanings as defined by the general formulas (D.I) of claim 24 and (D-II) of claim 25 and
**R**^{**8**} represents NYY¹, -O(R⁴O)ₓH or -O(R⁴O)ₓC(O)-CHR-CHR¹-C(O)NYY¹.

27. A surfactant composition according to any one of claims 1 to 13, **characterized in that** the gemini surfactant has the general formula (D.IV) 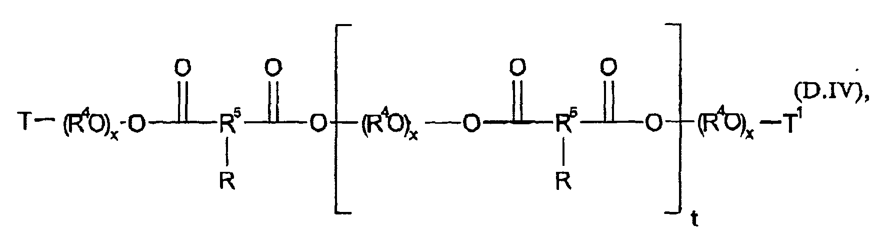 wherein the substituents have the meanings as defined by the general formula (D.I) of claim 24, (D.II) of claim 25, and (D.III) of claim 26, and
**t** is an integer from 1 to 100, preferably 1 to 20, most preferably 1 to 4.

28. A surfactant composition according to any one of claims 1 to 4, **characterized in that** the surfactant composition comprises a gemini surfactant of the general formula (AI) as component (A) and acyllactylate and/or acylglutamates as component (B).

29. A surfactant composition according to any one of claims 1 to 4, **characterized in that** the surfactant composition comprises a gemini surfactant of the general formula (AIII) as component (A) and acyllactylates and/or acylglutamates as component (B).

30. A surfactant composition according to any one of claims 1 to 4, **characterized in that** the surfactant composition comprises a gemini surfactant of the general formula (CII) as component (A) and acyllactylates and/or acylglutamates as component (B).

31. A surfactant composition according to any one of claims 1 to 4, **characterized in that** the surfactant composition comprises a gemini surfactant of the general formula (DI) as component (A) and acyllactylates and/or acylglutamates as component (B).

32. The use of the surfactant composition as claimed in any one of the preceding claims as a constituent of skin and hair cleaning preparations.

## Revendications

1. Composition tensioactive comprenant
(A) à raison de 1 à 90 % en poids, rapporté à la somme des composants (A) et (B), un ou plusieurs tensioactifs géminés et
(B) pour le reste, rapporté à la somme des composants (A) et (B), au moins un autre composant détergent avec une caractéristique de mauvais moussage,
le composant détergent contenant des lactylates d'acyle et/ou des glutamates d'acyle.

2. Composition tensioactive selon la revendication 1, **caractérisée en ce que** la composition tensioactive comprend
(A) à raison de 20 à 60 % en poids, rapporté à la somme des composants (A) et (B), un ou plusieurs tensioactifs géminés et
(B) pour le reste, rapporté à la somme des composants (A) et (B), un ou plusieurs composants détergents.

3. Composition tensioactive selon l'une quelconque des revendications précédentes contenant en outre
(C) à raison d'au moins 0,1 % en poids, rapporté à la composition totale, de l'eau.

4. Composition tensioactive selon l'une quelconque des revendications précédentes contenant en outre
(D) à raison d'au moins 0,1 % en poids, rapporté à la composition totale, un ou plusieurs composants d'huile.

5. Composition tensioactive selon l'une quelconque des revendications précédentes, **caractérisée en ce que** le composant détergent contient des lactylates d'acyle.

6. Composition tensioactive selon l'une quelconque des revendications précédentes, **caractérisée en ce que** celle-ci contient comme composant détergent des sels de sodium, potassium, magnésium ou calcium de l'acide lactique monomère estérifié sur le groupement hydroxyle avec des acides carboxyliques en C₆ à C₂₄ linéaires ou ramifiés, saturés ou insaturés 1 à 3 fois non successivement, cycliques ou acycliques, ou de ses oligomères, le degré d'oligomérisation de l'acide lactique se situant à 1,1 jusqu'à 10, de préférence à 1,1 jusqu'à 4.

7. Composition tensioactive selon l'une quelconque des revendications 1 à 4, **caractérisée en ce que** le composant détergent contient des glutamates d'acyle.

8. Composition tensioactive selon l'une quelconque des revendications 1 à 4, **caractérisée en ce que** les glutamates d'acyle sont des glutamates d'acyle présentant 6 à 24 atomes de carbone dans la chaîne acyle et la chaîne alcyle est linéaire ou ramifiée, saturée ou insaturée 1 à 3 fois non successivement.

9. Composition tensioactive selon l'une quelconque des revendications précédentes, **caractérisée en ce que** le tensioactif géminé présente des atomes d'azote à l'endroit de liaison entre l'espaceur, le groupement hydrophile et le groupement hydrophobe.

10. Composition tensioactive selon l'une quelconque des revendications précédentes, **caractérisée en ce que** le tensioactif géminé ou selon le cas son mélange présente un espaceur comportant 1 à 12 atomes de C et présentant des groupements amine ou amide.

11. Composition tensioactive selon l'une quelconque des revendications précédentes, **caractérisée en ce que** le tensioactif géminé présente en tant que groupement double hydrophobe à chaque fois un radical hydrocarboné en C₆ à C₂₄, et/ou en tant groupement (de tête) double hydrophile au moins un radical alcoxylé qui porte un groupement acide sulfonique, acide carboxylique, acide phosphonique, polyalcool ou poly(oxyde d'alkylène) qui le cas échéant peut également se présenter sous forme de sel.

12. Composition tensioactive selon l'une quelconque des revendications précédentes ou selon l'une quelconque des revendications 28 à 32, **caractérisée en ce que** le composant (A) est contenu dans la composition tensioactive à raison de 1 à 30 % en poids rapporté au total des tensioactifs ioniques, de préférence anioniques, qui ne sont pas des tensioactifs géminés comme le composant (A).

13. Composition tensioactive selon l'une quelconque des revendications précédentes ou selon l'une quelconque des revendications 28 à 31, **caractérisée en ce que** les composants (A) et (B) sont contenus au total à raison de 0,1 à 40 % en poids, de préférence à raison de 0,1 à 10 % en poids, dans la composition totale.

14. Composition tensioactive selon l'une quelconque des revendications 1 à 13, **caractérisée en ce que** le tensioactif géminé présente la formule générale (A.I) 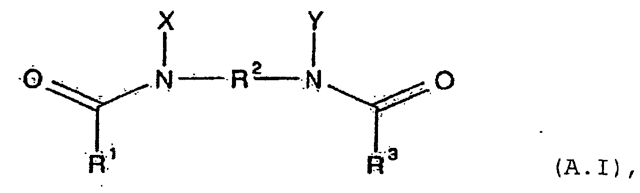 les substituants ayant la signification suivante :
R¹, R³ alkyle en C₅ à C₂₅, ramifié ou non ramifié, saturé, le cas échéant insaturé jusqu'à deux fois non successivement ;
R² alkylène en C₁ à C₁₂;
X, Y (C₂H₄O-)ₓ(C₃H₆O-)_{y}-FR ; x + y ≥ 1, x:0-15, y : 0-10 et
FR -SO₃M, -CH₂-CO₂M, -P (O) (OM)₂, H, -C₃H₆SO₃M, -CH₂ (CHOH) ₄CH₂OH dans la mesure où x + y = 0, M = alcali, ammonium d'alkyle, ammonium d'alcanol, H ou ½ alcalino-terreux.

15. Composition tensioactive selon l'une quelconque des revendications 1 à 13, **caractérisée en ce que** le tensioactif géminé présente la formule générale (A.II) 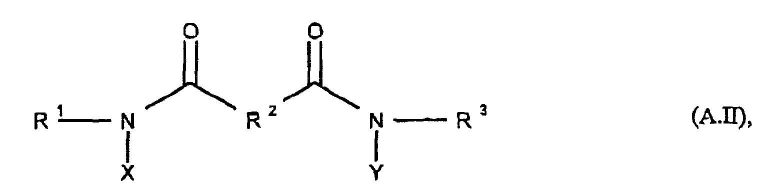 les substituants ayant la signification indiquée pour la formule générale (A.I) à la revendication 14.

16. Composition tensioactive selon l'une quelconque des revendications 1 à 13, **caractérisée en ce que** le tensioactif géminé présente la formule générale (A.III) 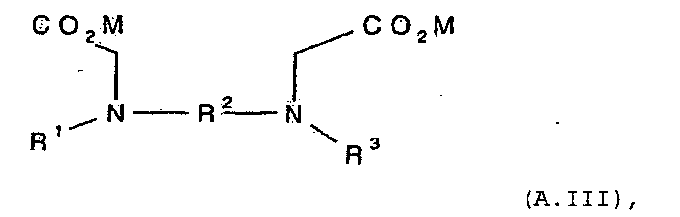 les substituants ayant la signification indiquée pour la formule générale (A.I) à la revendication 14.

17. Composition tensioactive selon l'une quelconque des revendications 1 à 13, **caractérisée en ce que** le tensioactif géminé présente la formule générale (B.I) 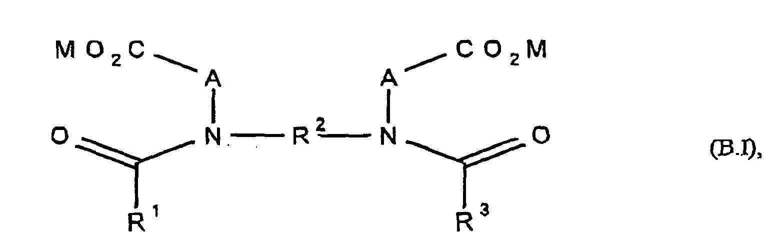 les substituants ayant la signification suivante :
R¹, R³ alkyle en C₅ à C₂₅, ramifié ou non ramifié, saturé, le cas échéant insaturé jusqu'à deux fois non successivement ;
R² alkylène en C₁ à C₁₂;
A CHR⁴, CH₂, C₂H₄, C₃H₆, C₄H₈;
R⁴ radical d'un acide aminocarboxylique et
M alcali, ammonium d'alkyle, ammonium d'alcanol, H ou ½ alcalino-terreux.

18. Composition tensioactive selon l'une quelconque des revendications 1 à 13, **caractérisée en ce que** le tensioactif géminé présente la formule générale (B.II) 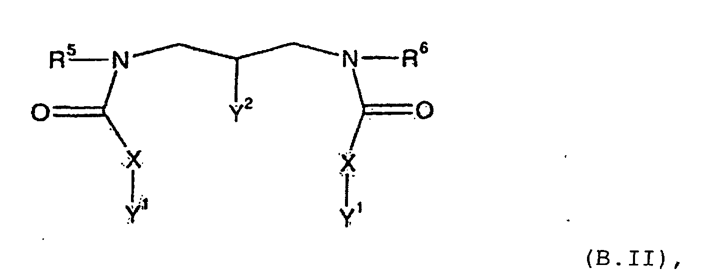 les substituants ayant la signification indiquée pour la formule générale (B.I) à la revendication 17 et
R⁵, R⁶ signifiant un alkyle en C₆ à C₃₆, ramifié ou non ramifié, saturé, le cas échéant insaturé jusqu'à deux fois non successivement ;
X signifiant un groupement alkylène ou alcénylène avec 1 à 6 atomes de carbone, qui le cas échéant est substitué par un groupement hydroxyle ou un groupement acide sulfonique ou un groupement carboxy ;
Y¹ signifiant un groupement sulfonate ou sulfate ou un groupement carboxyle et
Y² signifiant un groupement hydroxyle, un radical acide sulfurique ou -O-(CO)X-COOH.

19. Composition tensioactive selon l'une quelconque des revendications 1 à 13, **caractérisée en ce que** le tensioactif géminé présente la formule générale (B.III) 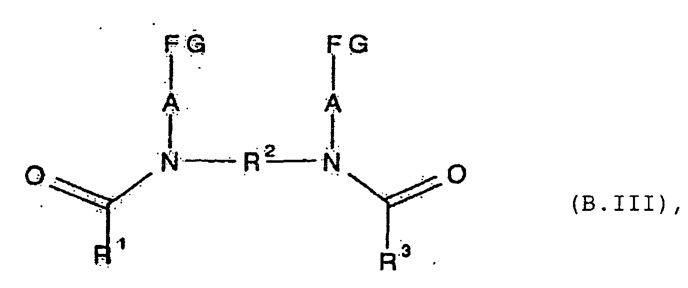 les substituants ayant la signification indiquée pour la formule générale (B.I) à la revendication 17 et
FG signifiant -COOM ou -SO₃M.

20. Composition tensioactive selon l'une quelconque des revendications 1 à 13, **caractérisée en ce que** le tensioactif géminé présente la formule générale (B.IV) 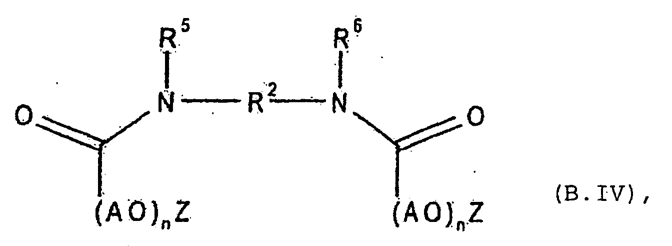 les substituants ayant la signification indiquée pour les formules générales B(I) à la revendication 17 et B(II) à la revendication 18 et
AO signifiant des unités d'oxyde d'alkylène, c'est-à-dire des unités éther d'éthylèneglycol, de propylèneglycol et de butylèneglycol, seules ou réparties statistiquement ou en blocs, avec n = 1 à 20 et
Z signifiant -SO₃M, -C₂H₄SO₃M, -C₃H₆SO₃M, -P(O) (OM)₂, -CH₂-COOM ou -C₂H₄-COOM.

21. Composition tensioactive selon l'une quelconque des revendications 1 à 13, **caractérisée en ce que** le tensioactif géminé présente la formule générale (C.I) 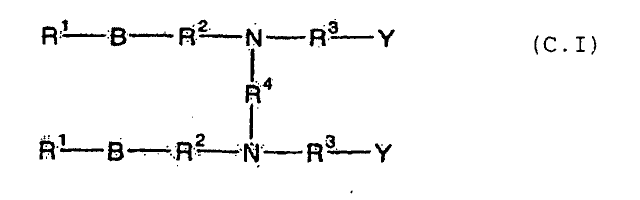 les substituants ayant la signification suivante :
R¹ est un alkyle en C₅ à C₂₅, ramifié ou non ramifié, saturé, le cas échéant insaturé jusqu'à 2 fois non successivement, substitué par hydroxy ou perfluoré;
R² est un alkylène en C₁ à C₁₂ ou des dérivés substitués par hydroxy de celui-ci ;
B est un groupement amide [-C(O)N(R²)- ou -N(R⁵)C(O)-], un groupement carboxyle [-C(O)O- ou -O(C(O)-], un groupement polyéther [O(R⁶-O)ₓ-] ;
R⁵ est un alkyle en C₁ à C₄ ou un alkyle substitué par hydroxy ou un H ;
R⁶ est un alkylène en C₂ à C₄ ;
x est un nombre de 1 à 20 ;
R³ est un alkyle en C₁ à C₁₂ ou des dérivés substitués par hydroxy de celui-ci, R⁷-D-R⁷ ou un groupement polyéther [-O(R⁶-O)ₓ-] ;
R⁷ est un alkylène en C₁ à C₆ ou des dérivés substitués par hydroxy de celui-ci ;
D est -O-, -S-, -N(R⁸)- ;
R⁴ est un alkylène ou alkylaryle avec 1 à 12 atomes de C ou les dérivés substitués par hydroxy ou R⁹-D¹-R⁹ ;
R⁸ est un alkyle en C₁ à C₃₂ ou un alkyle substitué par hydroxy ou un H ou un R⁹-D¹-R⁹;
R⁹ est un alkylène en C₁ à C₆ ou des dérivés substitués par hydroxy de celui-ci ou un aryle;
D¹ est -O-, -S-, -SO₂-, -C(O)-, [-O (R⁷-O)ₓ-], (R¹⁰)ₜ[N(R¹⁰)_{z}] ou aryle ;
R¹⁰ est un alkyle en C₁ à C₁₂ ou un alkyle substitué par hydroxy ou un H ou un aryle ;
t,z signifient indépendamment l'un de l'autre un nombre de 1 à 4 et
Y sont indépendamment l'un de l'autre -SO₃H, O-SO₃H, -OP(O) (OH)₂, -P(O) (OH)₂, -COOH, -CO₂-C₆H₄-SO₃H ou leurs sels.

22. Composition tensioactive selon l'une quelconque des revendications 1 à 13, **caractérisée en ce que** le tensioactif géminé présente la formule générale (C.II) 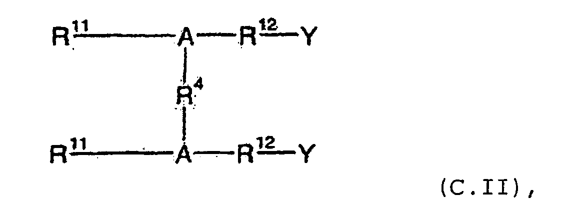 les substituants ayant la signification indiquée pour la formule générale (C.I) à la revendication 21 et
R¹¹ signifiant un alkyle en C₅ à C₂₃, ramifié ou non ramifié, saturé, le cas échéant insaturé jusqu'à 2 fois non successivement, substitué par hydroxy ou perfluoré ou R¹⁴-BR² ;
R¹⁴ signifiant un alkyle en C₁ à C₁₂, ramifié ou non ramifié, saturé, le cas échéant insaturé jusqu'à 2 fois non successivement, ou les dérivés substitués par hydroxy ;
R¹² signifiant un alkylène en C₁ à C₁₂, ramifié ou non ramifié, saturé, le cas échéant insaturé jusqu'à 2 fois non successivement, ou les dérivés substitués par hydroxy ou un groupement amide [-C(O)N(R²)- ou -N(R⁵)C(O)-], un groupement carboxylique [-C(O)O- ou -OC(O)-], un groupement polyether [-(O(R⁶-O)ₓ-] ou R⁹-D¹-R⁹ et
A signifiant -CR⁶= ou -N= à condition que si A est identique à -N=, R¹¹ est identique à R¹⁴-B-R².

23. Composition tensioactive selon l'une quelconque des revendications 1 à 13, **caractérisée en ce que** le tensioactif géminé présente la formule générale (C.III) 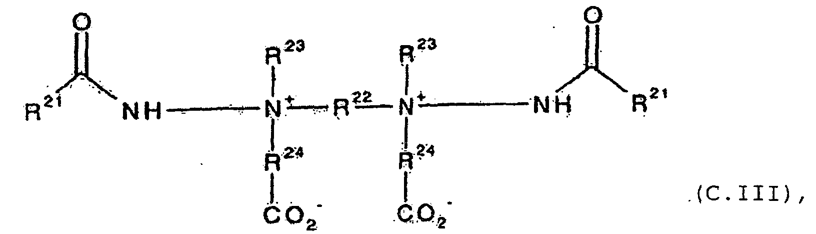 les substituants ayant la signification indiquée pour les formules générales (C.I) et (C.II) et
R²¹ signifiant un alkyle en C₅ à C₂₃, ramifié ou non ramifié, saturé, le cas échéant insaturé jusqu'à 2 fois non successivement ;
R²², R²⁴ signifiant un alkylène en C₁ à C₆
R²³ signifiant un méthyle, éthyle, propyle ou un groupement polyéther [-O(R⁶-O)ₓ-].

24. Composition tensioactive selon l'une quelconque des revendications 1 à 13, **caractérisée en ce que** le tensioactif géminé présente la formule générale (D.I) 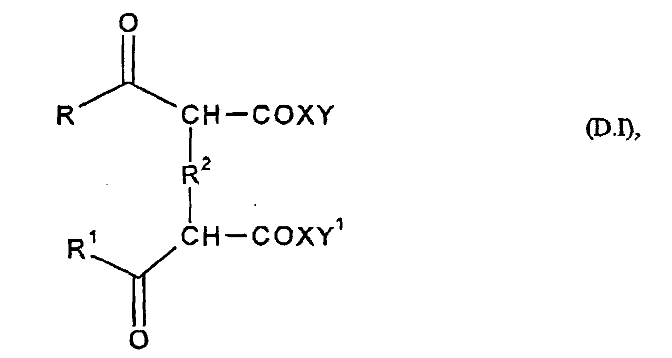 les substituants ayant la signification suivante :
R, R¹ est un alkyle en C₅ à C₃₀, ramifié ou non ramifié, saturé, le cas échéant insaturé jusqu'à 2 fois non successivement, substitué par hydroxy ou perfluoré ;
R² est un alkylène en C₁ à C₁₀, un arylène ou leurs dérivés substitués par hydroxy, un polyéther [-O(R⁴O)ₓ-], -S-, -SO₂-, -O-, -S-S-, -O-R⁵-O- ou -S-R⁵-S- ; variable pour une liaison directe entre les deux carbones en α ;
R⁴ est un alkylène en C₂ à C₄ ;
R⁵ est un alkylène en C₁ à C₁₀, un arylène ou un arylarylène, -N(R⁶)- ou un -(NR⁶)-R⁷-(NR⁶) - ;
R⁶ est un alkyle en C₁ à C₆ ;
R⁷ est un alkyle en C₁ à C₆, R⁷ et R⁶ pouvant être un noyau hétérocyclique ;
X est un polyéther [-O(R⁴O)ₓ-], x étant un nombre de 1 à 30, -O-, NZ ;
Z est un alkyle en C₁ à C₁₀, un aryle, un alkylaryle ou un H et
Y, Y¹ sont indépendamment l'un de l'autre H, -CH₂-COOH et sels, un radical hydrocarboné avec au moins deux groupements hydroxyle, tel que l'érythrose, le thréose, le ribose, l'arabinose, le xylose, le fructose, le lyxose, l'allose, l'altrose, le glucose, le mannose, le galactose et leurs mélanges.

25. Composition tensioactive selon l'une quelconque des revendications 1 à 13, **caractérisée en ce que** le tensioactif géminé présente la formule générale (D.II) 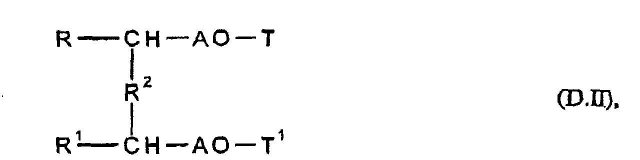 les substituants ayant la signification indiquée pour la formule générale (D.I) à la revendication 24 et
AO signifiant -C(O)-, -C (O)-[-O(R⁴O)ₓ-], -CH₂-[-O(R⁴O)ₓ-], -CH₂O-;
T, T¹ signifiant indépendamment l'un de l'autre -OM, -H, -CH₃, -C₂H₅, -SO₃M, -CH₂COOM, -C₂H₄-COOM, -C₃H₆-SO₃M, -O-P (O) (OM)₂ et
M signifiant un alcali, un ½ alcalino-terreux, un ammonium, un mono-, di-, trialcanolammonium ou un H.

26. Composition tensioactive selon l'une quelconque des revendications 1 à 13, **caractérisée en ce que** le tensioactif qéminé présente la formule générale (D.III) 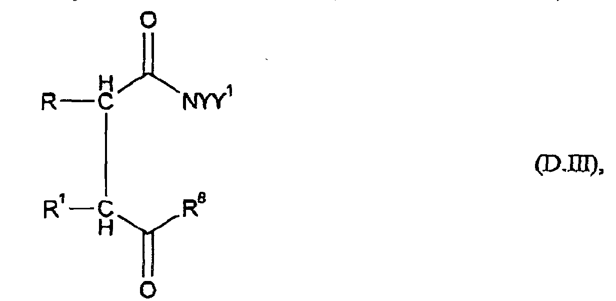 les substituants ayant la signification indiquée pour les formules générales (D.I) à la revendication 24 et (D.II) à la revendication 25 et
R⁸ signifiant NYY¹, -O(R⁴O)ₓH ou -O(R⁴O)ₓ-C(O)-CHR-CHR¹-C (O) NYY¹.

27. Composition tensioactive selon l'une quelconque des revendications 1 à 13, **caractérisée en ce que** le tensioactif géminé présente la formule générale (D.IV) 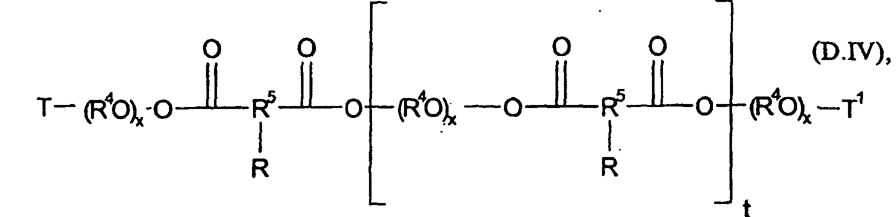 les substituants ayant la signification indiquée pour les formules générales (D.I) à la revendication 24 et (D.II) à la revendication 25 et (D.III) à la revendication 26 et t signifiant un nombre entier de 1 à 100, de préférence de 1 à 20, de façon particulièrement préférée de 1 à 4.

28. Composition tensioactive selon l'une quelconque des revendications 1 à 4, **caractérisée en ce que** la composition tensioactive contient en tant que composant (A) un tensioactif géminé de formule générale (AI) et en tant que composant (B) du lactylate d'acyle et/ou des glutamates d'acyle.

29. Composition tensioactive selon l'une quelconque des revendications 1 à 4, **caractérisée en ce que** la composition tensioactive contient en tant que composant (A) un tensioactif géminé de formule générale (AIII) et en tant que composant (B) des lactylates d'acyle et/ou des glutamates d'acyle.

30. Composition tensioactive selon l'une quelconque des revendications 1 à 4, **caractérisée en ce que** la composition tensioactive contient en tant que composant (A) un tensioactif géminé de formule générale (CII) et en tant que composant (B) des lactylates d'acyle et/ou des glutamate d'acyle.

31. Composition tensioactive selon l'une quelconque des revendications 1 à 4, **caractérisée en ce que** la composition tensioactive contient en tant que composant (A) un tensioactif géminé de formule générale (DI) et en tant que composant (B) des lactylates d'acyle et/ou des glutamates d'acyle.

32. Utilisation de la composition tensioactive selon l'une quelconque des revendications précédentes comme matière constitutive de formulations de produits de nettoyage des cheveux et de la peau.
